# EUROPEAN PATENT SPECIFICATION

(11) **EP 4 100 409 B1**
(45) Date of publication and mention of the grant of the patent: **28.08.2024**
(21) Application number: 21702989.1
(22) Date of filing: 01.02.2021
(51) Int. Cl.: C07D 487/04, A61K 31/519, A61P 35/00

(54) **[1,3]DIAZINO[5,4-D]PYRIMIDINES AS HER2 INHIBITORS**
[1,3]DIAZINO[5,4-D]PYRIMIDINE ALS HER2-INHIBITOREN
[1,3]DIAZINO[5,4-D]PYRIMIDINES EN TANT QU'INHIBITEURS DE HER2

(30) Priority: 03.02.2020 EP 20155157
(43) Date of publication of application: 14.12.2022
(73) Proprietor: Boehringer Ingelheim International GmbH, 55216 Ingelheim am Rhein (DE)
(72) Inventor: WILDING, Birgit, 55216 INGELHEIM AM RHEIN (DE); BOESE, Dietrich, 55216 INGELHEIM AM RHEIN (DE); ENGELHARDT, Harald, 55216 INGELHEIM AM RHEIN (DE); FUCHS, Julian, 55216 INGELHEIM AM RHEIN (DE); NEUMUELLER, Ralph, 55216 INGELHEIM AM RHEIN (DE); PETRONCZKI, Mark, 55216 INGELHEIM AM RHEIN (DE); SCHARN, Dirk, 55216 INGELHEIM AM RHEIN (DE); TREU, Matthias, 55216 INGELHEIM AM RHEIN (DE)
(74) Representative: Lutze, Oliver
(86) International application number: PCT/EP2021/052261
(87) International publication number: WO 2021/156178

(56) References cited:
- WO-A1-2019/042409
- WO-A2-2007/059257
- ZHANG YALING ET AL: "Enrichment of novel quinazoline derivatives with high antitumor activity in mitochondria tracked by its self-fluorescence", EUROPEAN JOURNAL OF MEDICINAL CHEMISTRY, ELSEVIER, AMSTERDAM, NL, vol. 178, 6 June 2019 (2019-06-06), pages 417 - 432, XP085751583, ISSN: 0223-5234, [retrieved on 20190606], DOI: 10.1016/J.EJMECH.2019.06.015

## Description

### Field of the invention

The present invention relates to [1,3]diazino[5,4-d]pyrimidines and derivatives of Formula **(I)** 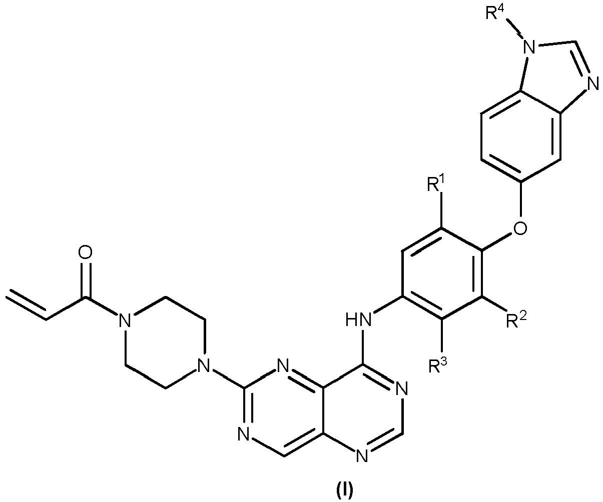 wherein the groups **R¹, R², R³** and **R⁴** have the meanings given in the claims and specification, their use as inhibitors of HER2 and its mutants, pharmaceutical compositions which contain such compounds and their use as medicaments, especially as agents for treatment and/or prevention of oncological diseases.

### Background of the invention

The family of ERBB transmembrane receptor tyrosine kinases (RTKs) consists of the four members EGFR (ERBB1), HER2 (Neu, ERBB2), HER3 (ERBB3) and HER4 (ERBB4), which fulfill essential functions during development (Citri et al., Nat. Rev. Mol. Cell. Biol., 2006, 7(7), 505-516; Hynes et al., Curr. Opin. Cell. Biol., 2009, 21(2), 177-184; Wang, Z., Methods Mol. Biol., 2017, 1652, 3-35). ERBB signaling is initiated upon binding of the extracellular domains of EGFR, HER3 or HER4 to their respective ligands and subsequent homo- or heterodimerization of ERBB family members. HER2, for which no ligand has been identified, is the preferred dimerization partner for the other ERBB members. Once an active ligand-receptor complex has been formed, the intracellular tyrosine kinase domains of EGFR, HER2 or HER4 are activated by auto- or transphosphorylation and subsequently elicit a signal transduction cascade most notably engaging the mitogen-activated protein (MAP) kinase and/or the phosphoinositide 3-kinase (PI3K) pathways (Citri et al, Nat. Rev. Mol. Cell. Biol., 2006, 7(7), 505-516; Hynes et al., Curr. Opin. Cell. Biol., 2009, 21(2), 177-184; Wang, Z., Methods Mol. Biol., 2017, 1652, 3-35.).

Aberrant ERBB signaling is implicated in several pathophysiological conditions including cancer or neurological diseases. In cancer, ERBB signaling is hyper-activated through mutations that render the RTK constitutively active by promoting dimerization or shifting the equilibrium towards the active conformer of the kinase and/or through amplification and consequent over-expression of the RTK. Both oncogenic mechanisms increase the net output of ERBB signaling and thereby promote cell survival, cell growth and proliferation (Arteaga et al., Cancer Cell, 2014, 25(3), 282-303).

Aberrant HER2 signaling is observed in a wide variety of human malignancies. Oncogenic mutations are described for the extracellular, (juxta-) membrane and intracellular regions of the protein. Collectively these mutations render HER2 constitutively active, fueling cancer initiation, tumor maintenance and growth (Connell et al., ESMO Open, 2017, 2(5), e000279). Similarly, HER2 overexpression increases HER2 signaling and underlies neoplastic transformation and tumor maintenance in a variety of indications including breast, gastric or lung cancer.

Consequently, interference with HER2 oncogenic signaling results in inhibition of tumor growth. Targeted therapies include HER2 directed antibodies (including trastuzumab and pertuzumab), HER2 directed antibody-drug conjugates (trastuzumab-DM1 (T-DM1, ado-trastuzumab emtansine)) and small molecules inhibiting the HER2 kinase domain (afatinib, neratinib, lapatinib).

Altogether, tumors driven by HER2 oncogenic mutations or HER2 wild type amplification might benefit from a HER2 specific tyrosine kinase inhibitor (TKI). Collectively, HER2 alterations affect up to 6-7% of all human cancers and an EGFR wild type sparing TKI (tyrosine kinase inhibitor) could emerge as an effective therapeutic option.

Even though there are HER2 wild type inhibitors that are selective over EGFR wild type, such as tucatinib, these inhibitors do not have efficacy on HER2 carrying exon 20 mutations. Other selective wild type HER2 inhibitors have been disclosed in prior art documents WO 2003/049740, WO 2007/059257, WO 2005/044302.

HER2 exon 20 mutations constitute a subset of HER2 gain-of-function mutations that result in enhanced kinase activity (Wang et al. Cancer Cell, 2006, 10(1): 25-38). This enhanced HER2 kinase activity feeds into downstream signaling cascades that stimulate neoplastic transformation through promoting growth, proliferation and survival of the mutant cells.

Studies in genetically engineered mouse models have demonstrated that the most prevalent HER2 exon 20 mutation in NSCLC, the duplication of the 4 amino acids YVMA (p.A775_G776insYVMA), is required and sufficient to drive oncogenic growth (Perera et al., Proc. Natl. Acad. Sci. USA, 2009, 106(2), 474-479). Withdrawal of HER2-YVMA expression is associated with tumor shrinkage, suggesting that this oncogenic variant of HER2 is required for tumor maintenance (Perera et al. 2009). In addition, this study demonstrated that in a mouse model, the pan-ERBB blocker Afatinib, is efficacious in vivo and can interfere with oncogenic signaling of HER2-YVMA (Perera et al. 2009).

Oncogenic mutations in HER2 in NSCLC predominantly affect the tyrosine kinase domain of HER2 and cluster in exon 20 of the ERBB2 gene (Stephens et al., Nature, 2004, 431(7008), 525-6). 2-4% of lung cancer patients are estimated to carry activating mutations in HER2 exon 20. Clinically approved ERBB targeting tyrosine kinase inhibitors are not efficacious in these patients, as they are limited by EGFR wild type-mediated dose limiting toxicity. Afatinib and other pan-ERBB blockers have shown limited efficacy in HER2 exon 20 mutated NSCLC patients, mainly due to limitations in reaching an efficacious dose. In particular, EGFR wild type mediated toxicity limits efficacious dosing.

Allitinib, ibrutinib, neratinib, poziotinib and pyrotinib are known pan-ERBB inhibitors of mutant HER2 exon 20. Further inhibitors of mutant HER2 exon 20 have been disclosed in prior art documents WO 2015/175632, WO 2015/195228, WO 2016/183278 and WO 2019/046775.

There is a high unmet medical need for compounds that selectively target HER2 exon 20 mutant proteins while sparing EGFR wild type to overcome the disadvantages of EGFR wild type mediated dose limiting toxicity.

It has been found that the compounds of the present invention bind to the tyrosine kinase domain of wild type and mutant HER2 exon 20 in an orthosteric and covalent manner while sparing EGFR wild type and act as selective inhibitors of wild type HER2 and mutant HER2 carrying mutations in exon 20.

### Summary of the invention

The aim of the present invention is to provide inhibitors of mutant HER2 exon 20 that are selective over EGFR wild type. The compounds of the invention act as selective inhibitors of HER2 exon 20 and show an improved wild type EGFR sparing efficacy profile in addition to high selectivity over EGFR wild type, compared to prior art compounds. Furthermore, some compounds of the present invention show an improved pharmacokinetic and pharmacological profile, such as good metabolic stability.

The compounds of the invention are useful for the prevention and/or treatment of a disease and/or condition characterised by excessive or abnormal cell proliferation, especially in the treatment and/or prevention of cancer.

### Detailed description of the Invention

The present invention relates to new [1,3]diazino[5,4-d]pyrimidines and derivatives of Formula **(I)** 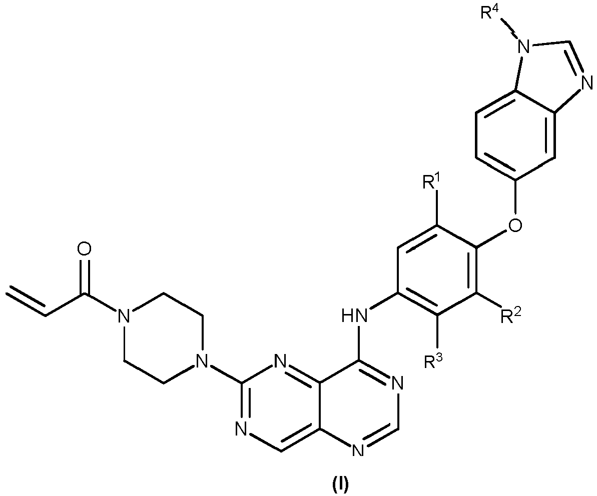 wherein
***(A0)***
   **R¹** is selected from the group consisting of hydrogen, -CH₃, -CCH, -OCH₃ and halogen
***(B0)***
   **R²** is hydrogen or halogen
***(C0)***
   **R³** is hydrogen or halogen
***(D0)***
   **R⁴** is hydrogen or -CH₃
   and at least one of **R¹, R²** and **R³** is not hydrogen
   or a salt thereof.

In another embodiment **(A1)** the invention relates to compounds of Formula **(I)**, wherein **R¹** is selected from the group consisting of -CH₃, -CCH, -OCH₃ and halogen
or a salt thereof.

In another embodiment **(A2)** the invention relates to compounds of Formula **(I)**, wherein **R¹** is selected from the group consisting of -CH₃, -CCH, -OCH₃, chlorine, bromine and fluorine
or a salt thereof.

In another embodiment **(A3)** the invention relates to compounds of Formula **(I)**, wherein **R¹** is hydrogen
or a salt thereof.

In another embodiment **(A4)** the invention relates to compounds of Formula **(I)**, wherein **R¹** is -CH₃
or a salt thereof.

In another embodiment **(A5)** the invention relates to compounds of Formula **(I)**, wherein **R¹** is -CCH
or a salt thereof.

In another embodiment **(A6)** the invention relates to compounds of Formula **(I)**, wherein **R¹** is -OCH₃
or a salt thereof.

In another embodiment **(A7)** the invention relates to compounds of Formula **(I)**, wherein **R¹** is halogen
or a salt thereof.

In another embodiment **(A8)** the invention relates to compounds of Formula **(I)**, wherein **R¹** is selected from the group consisting of chlorine, bromine and fluorine
or a salt thereof.

In another embodiment **(A9)** the invention relates to compounds of Formula **(I),** wherein **R¹** is chlorine
or a salt thereof.

In another embodiment **(A10)** the invention relates to compounds of Formula **(I),** wherein **R¹** is bromine
or a salt thereof.

In another embodiment **(A11)** the invention relates to compounds of Formula **(I),** wherein **R¹** is fluorine
or a salt thereof.

In another embodiment **(B1)** the invention relates to compounds of Formula **(I),** wherein **R²** is hydrogen
or a salt thereof.

In another embodiment **(B2)** the invention relates to compounds of Formula **(I),** wherein **R²** is halogen
or a salt thereof.

In another embodiment **(B3)** the invention relates to compounds of Formula **(I),** wherein **R²** is selected from the group consisting of chlorine, bromine and fluorine
or a salt thereof.

In another embodiment **(B4)** the invention relates to compounds of Formula **(I),** wherein **R²** is chlorine
or a salt thereof.

In another embodiment **(B5)** the invention relates to compounds of Formula **(I),** wherein **R²** is bromine
or a salt thereof.

In another embodiment **(B6)** the invention relates to compounds of Formula **(I)**, wherein **R²** is fluorine
or a salt thereof.

In another embodiment **(C1)** the invention relates to compounds of Formula **(I)**, wherein **R³** is hydrogen
or a salt thereof.

In another embodiment **(C2)** the invention relates to compounds of Formula **(I)**, wherein **R³** is halogen
or a salt thereof.

In another embodiment **(C3)** the invention relates to compounds of Formula **(I)**, wherein **R³** is selected from the group consisting of chlorine, bromine and fluorine
or a salt thereof.

In another embodiment **(C4)** the invention relates to compounds of Formula **(I)**, wherein **R³** is chlorine
or a salt thereof.

In another embodiment **(C5)** the invention relates to compounds of Formula **(I)**, wherein **R³** is fluorine
or a salt thereof.

In another embodiment **(C6)** the invention relates to compounds of Formula **(I)**, wherein **R³** is chlorine, fluorine or hydrogen
or a salt thereof.

In another embodiment **(D1)** the invention relates to compounds of Formula **(I)**, wherein **R⁴** is hydrogen
or a salt thereof.

In another embodiment **(D2)** the invention relates to compounds of Formula **(I)**, wherein **R⁴** is -CH₃
or a salt thereof.

Above mentioned individual embodiments represent structural subgroups **(A0)** to **(A11), (B0)** to **(B6), (C0)** to **(C6)** and **(D0)** to **(D2)** of compounds of Formula **(I)**. These structural subgroups may be combined with each other as **(A)(B)(C)(D)** to specifically define further embodiments of the invention of compounds of Formula **(I)**. Accordingly, further embodiments of the invention are herein defined as compounds of Formula **(I)**, as a combination of specific structural subgroups **(A)(B)(C)(D)** wherein **(A)** is selected from **(A0)** to **(A11**); **(B)** is selected from **(B0)** to **(B6); (C)** is selected from **(C0)** to **(C6)** and **(D)** is selected from **(D0)** to **(D2).**

Herein are disclosed hydrates, solvates, polymorphs, metabolites and prodrugs of compounds of Formula **(I)**.

In another embodiment the invention relates to a pharmaceutically acceptable salt of a compound of Formula **(I)**.

A preferred embodiment of the current invention are the above compounds of Formula (I), selected from the group consisting of examples I-01 to I-12.

| | |
|---|---|
| **I-01** | 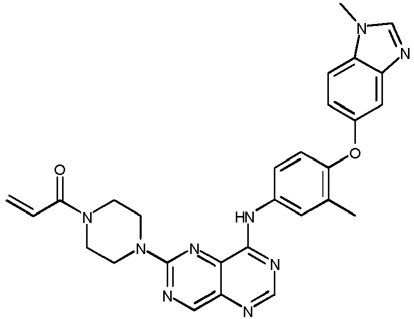 |
| **I-02** | 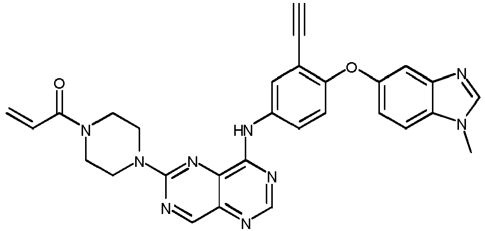 |
| **I-03** | 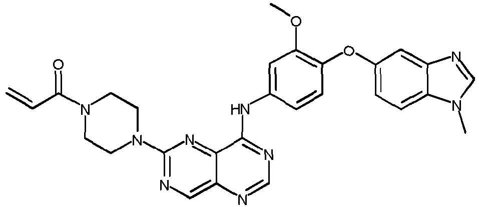 |
| **I-04** | 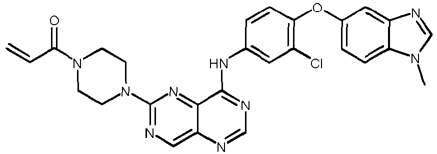 |
| **I-05** | 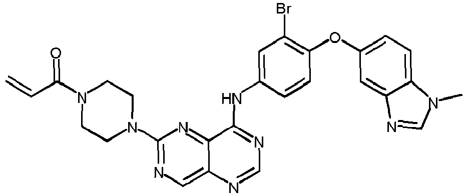 |
| **I-06** | 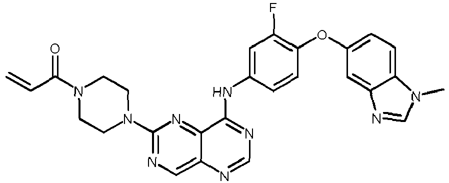 |
| **I-07** | 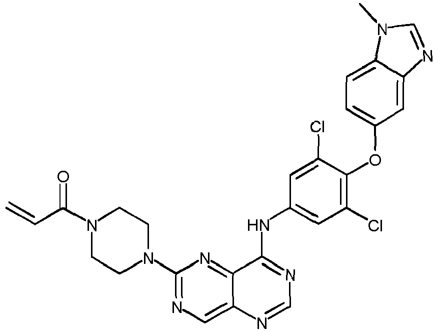 |
| **I-08** | 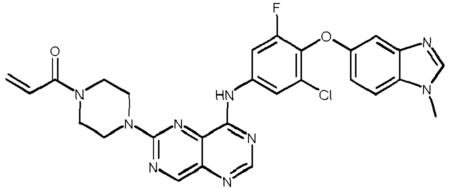 |
| **I-09** | 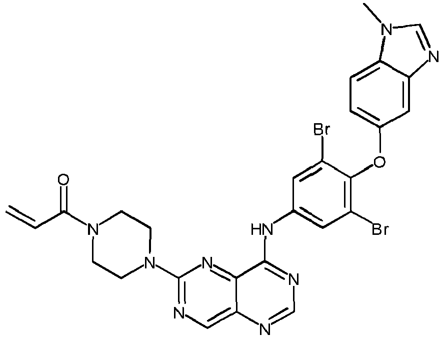 |
| **I-10** | 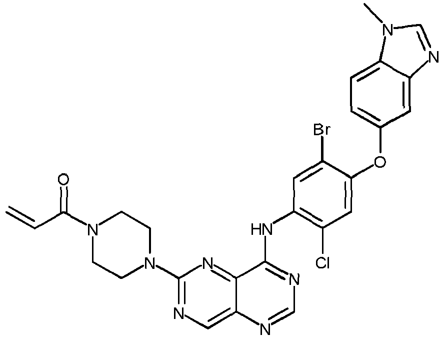 |
| **I-11** | 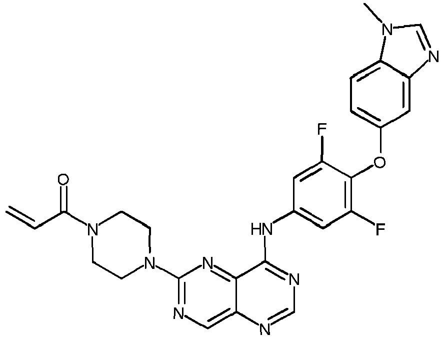 |
| **I-12** | 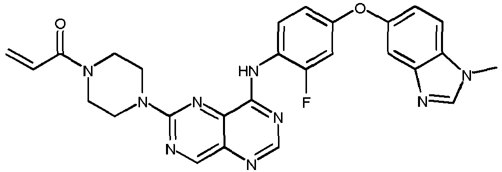 |

A further preferred embodiment of the current invention are pharmaceutically acceptable salts of the above compounds of Formula **(I),** selected from the group consisting of examples I-01 to I-12.

A further preferred embodiment of the current invention is the compound of example I-01. A further preferred embodiment of the current invention is the compound of example I-02. A further preferred embodiment of the current invention is the compound of example I-03. A further preferred embodiment of the current invention is the compound of example I-04. A further preferred embodiment of the current invention is the compound of example I-05. A further preferred embodiment of the current invention is the compound of example I-06. A further preferred embodiment of the current invention is the compound of example I-07. A further preferred embodiment of the current invention is the compound of example I-08. A further preferred embodiment of the current invention is the compound of example I-09. A further preferred embodiment of the current invention is the compound of example I-10. A further preferred embodiment of the current invention is the compound of example I-11. A further preferred embodiment of the current invention is the compound of example I-12.

A further preferred embodiment of the current invention are pharmaceutically acceptable salts of the compound of example I-01.

A further preferred embodiment of the current invention are pharmaceutically acceptable salts of the compound of example I-02.

A further preferred embodiment of the current invention are pharmaceutically acceptable salts of the compound of example I-03.

A further preferred embodiment of the current invention are pharmaceutically acceptable salts of the compound of example I-04.

A further preferred embodiment of the current invention are pharmaceutically acceptable salts of the compound of example I-05.

A further preferred embodiment of the current invention are pharmaceutically acceptable salts of the compound of example I-06.

A further preferred embodiment of the current invention are pharmaceutically acceptable salts of the compound of example I-07.

A further preferred embodiment of the current invention are pharmaceutically acceptable salts of the compound of example I-08.

A further preferred embodiment of the current invention are pharmaceutically acceptable salts of the compound of example I-09.

A further preferred embodiment of the current invention are pharmaceutically acceptable salts of the compound of example I-10.

A further preferred embodiment of the current invention are pharmaceutically acceptable salts of the compound of example I-11.

A further preferred embodiment of the current invention are pharmaceutically acceptable salts of the compound of example I-12.

The references to methods of treatment in subsequent paragraphs of this description are to be interpreted as reference to the compounds, pharmaceutical compositions and medicaments of the present invention for use in a method for treatment of the human or animal body by therapy or for diagnosis.

In another aspect, the disclosure provides a method of inhibiting wild type and/or mutant HER2 in a cell, comprising contacting the cell with a compound of Formula **(I)**. In another embodiment, the disclosure provides a method of inhibiting HER2 carrying exon 20 mutations in a cell, preferably comprising contacting the cell with a compound of Formula **(I)**.

In another aspect, the disclosure provides a method of inhibiting phosphorylation of wild type and/or mutant HER2 in a cell, comprising contacting the cell with a compound of Formula **(I)**. In another embodiment, the disclosure provides a method of inhibiting phosphorylation of HER2 exon 20 mutant in a cell, comprising contacting the cell with a compound of Formula **(I)**.

In another aspect, the disclosure provides the use of a compound of Formula **(I)** - or a pharmaceutically acceptable salt thereof - for the treatment and/or prevention of a disease and/or condition, wherein the inhibition of wild type and/or mutant HER2 is of therapeutic benefit. In another embodiment, the disclosure provides the use of a compound of Formula **(I)** - or a pharmaceutically acceptable salt thereof - for the treatment and/or prevention of a disease and/or condition, wherein the inhibition of HER2 exon 20 mutant protein is of therapeutic benefit.

In another aspect, the disclosure provides a compound of Formula **(I)** - or a pharmaceutically acceptable salt thereof - for use in a method for inhibiting wild type and/or mutant HER2, in a human subject in need thereof, comprising administering to the subject a therapeutically effective amount of compound of Formula **(I)** - or a pharmaceutically acceptable salt thereof. In another embodiment, the disclosure provides a compound of Formula **(I)** - or a pharmaceutically acceptable salt thereof - for use in a method for inhibiting HER2 exon 20 mutant, in a human subject in need thereof, comprising administering to the subject a therapeutically effective amount of compound of Formula **(I)** - or a pharmaceutically acceptable salt thereof. **→ p. 12-A**

In another aspect, the invention relates a compound of Formula **(I)** - or a pharmaceutically acceptable salt thereof - for use as a medicament.

In another aspect, the disclosure provides a compound of Formula **(I)**, - or a pharmaceutically acceptable salt thereof - for the treatment and/or prevention of cancer. In another embodiment, the disclosure provides a compound of Formula **(I)** - or a pharmaceutically acceptable salt thereof - for the treatment and/or prevention of cancer, wherein the cancer is with HER2 overexpression and/or HER2 amplification. In another embodiment, the disclosure provides a compound of Formula **(I)** - or a pharmaceutically acceptable salt thereof - for the treatment and/or prevention of cancer, wherein the cancer is HER2 exon 20 mutant cancer. In another embodiment, the disclosure provides a compound of Formula **(I)** - or a pharmaceutically acceptable salt thereof - for the treatment and/or prevention of cancer, wherein the HER2 overexpressed, HER2 amplified and/or HER2 exon 20 mutant cancer is selected from brain cancer, breast cancer, biliary cancer, bladder cancer, cervical cancer, colorectal cancer, endometrial cancer, skin cancer, esophagus tumor, head and neck tumor, gastrointestinal cancer, gallbladder tumor, kidney cancer, liver cancer, lung cancer and prostate cancer.

In another aspect, the disclosure provides a method of treating and/or preventing above mentioned diseases and conditions comprising administering a therapeutically effective amount of a compound of Formula **(I)** - or a pharmaceutically acceptable salt thereof - to a human.

In another aspect, the disclosure provides a compound of Formula **(I)** - or a pharmaceutically acceptable salt thereof - for use in the treatment and/or prevention of above mentioned diseases and conditions.

In another aspect, the disclosure provides the use of a compound of Formula **(I)** - or a pharmaceutically acceptable salt thereof - for the preparation of a medicament for the treatment and/or prevention of above mentioned diseases and conditions.

Furthermore, the following cancers, tumors and other proliferative diseases may be treated with compounds of the invention, without being restricted thereto:
Cancers/tumors/carcinomas of the head and neck: e.g. tumors/carcinomas/cancers of the nasal cavity, paranasal sinuses, nasopharynx, oral cavity (including lip, gum, alveolar ridge, retromolar trigone, floor of mouth, tongue, hard palate, buccal mucosa), oropharynx (including base of tongue, tonsil, tonsillar pilar, soft palate, tonsillar fossa, pharyngeal wall), middle ear, larynx (including supraglottis, glottis, subglottis, vocal cords), hypopharynx, salivary glands (including minor salivary glands);
cancers/tumors/carcinomas of the lung: e.g. non-small cell lung cancer (NSCLC) (squamous cell carcinoma, spindle cell carcinoma, adenocarcinoma, large cell carcinoma clear cell carcinoma, bronchioalveolar), small cell lung cancer (SCLC) (oat cell cancer, intermediate cell cancer, combined oat cell cancer);
neoplasms of the mediastinum: e.g. neurogenic tumors (including neurofibroma, neurilemoma, malignant schwannoma, neurosarcoma, ganglioneuroblastoma, ganglioneuroma, neuroblastoma, pheochromocytoma, paraganglioma), germ cell tumors (including seminoma, teratoma, non-seminoma), thymic tumors (including thymoma, thymolipoma, thymic carcinoma, thymic carcinoid), mesenchymal tumors (including fibroma, fibrosarcoma, lipoma, liposarcoma, myxoma, mesothelioma, leiomyoma, leiomyosarcoma, rhabdomyosarcoma, xanthogranuloma, mesenchymoma, hemangioma, hemangioendothelioma, hemangiopericytoma, lymphangioma, lymphangiopericytoma, lymphangiomyoma);
cancers/tumors/carcinomas of the gastrointestinal (GI) tract: e.g. tumors/carcinomas/ cancers of the esophagus, stomach (gastric cancer), pancreas, liver and biliary tree (including hepatocellular carcinoma (HCC), e.g. childhood HCC, fibrolamellar HCC, combined HCC, spindle cell HCC, clear cell HCC, giant cell HCC, carcinosarcoma HCC, sclerosing HCC; hepatoblastoma; cholangiocarcinoma; cholangiocellular carcinoma; hepatic cystadenocarcinoma; angiosarcoma, hemangioendothelioma, leiomyosarcoma, malignant schwannoma, fibrosarcoma, Klatskin tumor), gall bladder, extrahepatic bile ducts, small intestine (including duodenum, jejunum, ileum), large intestine (including cecum, colon, rectum, anus; colorectal cancer, gastrointestinal stroma tumor (GIST)), genitourinary system (including kidney, e.g. renal pelvis, renal cell carcinoma (RCC), nephroblastoma (Wilms' tumor), hypernephroma, Grawitz tumor; ureter; urinary bladder, e.g. urachal cancer, urothelial cancer; urethra, e.g. distal, bulbomembranous, prostatic; prostate (androgen dependent, androgen independent, castration resistant, hormone independent, hormone refractory), penis);
cancers/tumors/carcinomas of the testis: e.g. seminomas, non-seminomas;
Gynecologic cancers/tumors/carcinomas: e.g. tumors/carcinomas/cancers of the ovary, fallopian tube, peritoneum, cervix, vulva, vagina, uterine body (including endometrium, fundus);
cancers/tumors/carcinomas of the breast: e.g. mammary carcinoma (infiltrating ductal, colloid, lobular invasive, tubular, adenocystic, papillary, medullary, mucinous), hormone receptor positive breast cancer (estrogen receptor positive breast cancer, progesterone receptor positive breast cancer), HER2 positive breast cancer, triple negative breast cancer, Paget's disease of the breast;
cancers/tumors/carcinomas of the endocrine system: e.g. tumors/carcinomas/cancers of the endocrine glands, thyroid gland (thyroid carcinomas/tumors; papillary, follicular, anaplastic, medullary), parathyroid gland (parathyroid carcinoma/tumor), adrenal cortex (adrenal cortical carcinoma/tumors), pituitary gland (including prolactinoma, craniopharyngioma), thymus, adrenal glands, pineal gland, carotid body, islet cell tumors, paraganglion, pancreatic endocrine tumors (PET; nonfluorineunctional PET, PPoma, gastrinoma, insulinoma, VIPoma, glucagonoma, somatostatinoma, GRFoma, ACTHoma), carcinoid tumors;
sarcomas of the soft tissues: e.g. fibrosarcoma, fibrous histiocytoma, liposarcoma, leiomyosarcoma, rhabdomyosarcoma, angiosarcoma, lymphangiosarcoma, Kaposi's sarcoma, glomus tumor, hemangiopericytoma, synovial sarcoma, giant cell tumor of tendon sheath, solitary fibrous tumor of pleura and peritoneum, diffuse mesothelioma, malignant peripheral nerve sheath tumor (MPNST), granular cell tumor, clear cell sarcoma, melanocytic schwannoma, plexosarcoma, neuroblastoma, ganglioneuroblastoma, neuroepithelioma, extraskeletal Ewing's sarcoma, paraganglioma, extraskeletal chondrosarcoma, extraskeletal osteosarcoma, mesenchymoma, alveolar soft part sarcoma, epithelioid sarcoma, extrarenal rhabdoid tumor, desmoplastic small cell tumor;
sarcomas of the bone: e.g. myeloma, reticulum cell sarcoma, chondrosarcoma (including central, peripheral, clear cell, mesenchymal chondrosarcoma), osteosarcoma (including parosteal, periosteal, high-grade surface, small cell, radiation-induced osteosarcoma, Paget's sarcoma), Ewing's tumor, malignant giant cell tumor, adamantinoma, (fibrous) histiocytoma, fibrosarcoma, chordoma, small round cell sarcoma, hemangioendothelioma, hemangiopericytoma, osteochondroma, osteoid osteoma, osteoblastoma, eosinophilic granuloma, chondroblastoma;
mesothelioma: e.g. pleural mesothelioma, peritoneal mesothelioma;
cancers of the skin: e.g. basal cell carcinoma, squamous cell carcinoma, Merkel's cell carcinoma, melanoma (including cutaneous, superficial spreading, lentigo maligna, acral lentiginous, nodular, intraocular melanoma), actinic keratosis, eyelid cancer;
neoplasms of the central nervous system and brain: e.g. astrocytoma (cerebral, cerebellar, diffuse, fibrillary, anaplastic, pilocytic, protoplasmic, gemistocytary), glioblastoma, gliomas, oligodendrogliomas, oligoastrocytomas, ependymomas, ependymoblastomas, choroid plexus tumors, medulloblastomas, meningiomas, schwannomas, hemangioblastomas, hemangiomas, hemangiopericytomas, neuromas, ganglioneuromas, neuroblastomas, retinoblastomas, neurinomas (e.g. acoustic), spinal axis tumors;
lymphomas and leukemias: e.g. B-cell non-Hodgkin lymphomas (NHL) (including small lymphocytic lymphoma (SLL), lymphoplasmacytoid lymphoma (LPL), mantle cell lymphoma (MCL), follicular lymphoma (FL), diffuse large cell lymphoma (DLCL), Burkitt's lymphoma (BL)), T-cell non-Hodgkin lymphomas (including anaplastic large cell lymphoma (ALCL), adult T-cell leukemia/lymphoma (ATLL), cutaneous T-cell lymphoma (CTCL), peripheral T-cell lymphoma (PTCL)), lymphoblastic T-cell lymphoma (T-LBL), adult T-cell lymphoma, lymphoblastic B-cell lymphoma (B-LBL), immunocytoma, chronic B-cell lymphocytic leukemia (BchlorineL), chronic T-cell lymphocytic leukemia (TchlorineL) B-cell small lymphocytic lymphoma (B-SLL), cutaneous T-cell lymphoma (CTLC), primary central nervous system lymphoma (PCNSL), immunoblastoma, Hodgkin's disease (HD) (including nodular lymphocyte predominance HD (NLPHD), nodular sclerosis HD (NSHD), mixed-cellularity HD (MCHD), lymphocyte-rich classic HD, lymphocyte-depleted HD (LDHD)), large granular lymphocyte leukemia (LGL), chronic myelogenous leukemia (CML), acute myelogenous/myeloid leukemia (AML), acute lymphatic/lymphoblastic leukemia (ALL), acute promyelocytic leukemia (APL), chronic lymphocytic/lymphatic leukemia (CLL), prolymphocytic leukemia (PLL), hairy cell leukemia, chronic myelogenous/myeloid leukemia (CML), myeloma, plasmacytoma, multiple myeloma (MM), plasmacytoma, myelodysplastic syndromes (MDS), chronic myelomonocytic leukemia (CMML);
cancers of unknown primary site (CUP).

All cancers/tumors/carcinomas mentioned above which are characterized by their specific location/origin in the body are meant to include both the primary tumors and the metastatic tumors derived therefrom.

All cancers/tumors/carcinomas mentioned above may be further differentiated by their histopathological classification:
Epithelial cancers, e.g. squamous cell carcinoma (SCC) (carcinoma in situ, superficially invasive, verrucous carcinoma, pseudosarcoma, anaplastic, transitional cell, lymphoepithelial), adenocarcinoma (AC) (well-differentiated, mucinous, papillary, pleomorphic giant cell, ductal, small cell, signet-ring cell, spindle cell, clear cell, oat cell, colloid, adenosquamous, mucoepidermoid, adenoid cystic), mucinous cystadenocarcinoma, acinar cell carcinoma, large cell carcinoma, small cell carcinoma, neuroendocrine tumors (small cell carcinoma, paraganglioma, carcinoid); oncocytic carcinoma;
Nonepithilial cancers, e.g. sarcomas (fibrosarcoma, chondrosarcoma, rhabdomyosarcoma, leiomyosarcoma, hemangiosarcoma, giant cell sarcoma, lymphosarcoma, fibrous histiocytoma, liposarcoma, angiosarcoma, lymphangiosarcoma, neurofibrosarcoma), lymphoma, melanoma, germ cell tumors, hematological neoplasms, mixed and undifferentiated carcinomas.

In another aspect not explicitly claimed, the present invention relates to a compound of Formula **(I)** - or a pharmaceutically acceptable salt thereof - for use in the treatment and/or prevention of cancer, wherein the compound is to be administered in combination with a cytostatic and/or cytotoxic active substance and/or in combination with radiotherapy and/or immunotherapy.

In another aspect not explicitly claimed, the present invention relates to a combination of compound of Formula **(I)** - or a pharmaceutically acceptable salt thereof - with a cytostatic and/or cytotoxic active substance and/or in combination with radiotherapy and/or immunotherapy for use in the treatment and/or prevention of cancer.

In another aspect not explicitly claimed, the present invention relates to a method of treating and/or preventing cancer, wherein said method comprises administering a compound of Formula **(I)** - or a pharmaceutically acceptable salt thereof - in combination with a cytostatic and/or cytotoxic active substance and/or in combination with radiotherapy and/or immunotherapy.

The compounds of the invention may be used on their own or in combination with one or several other pharmacologically active substances such as state-of-the-art or standard-of-care compounds, such as e.g. cell proliferation inhibitors, anti-angiogenic substances, steroids or immune modulators/checkpont inhibitors, and the like.

Pharmacologically active substances which may be administered in combination with the compounds according to the invention, include, without being restricted thereto, hormones, hormone analogues and antihormones (e.g. tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxyprogesterone, octreotide), aromatase inhibitors (e.g. anastrozole, letrozole, liarozole, vorozole, exemestane, atamestane), LHRH agonists and antagonists (e.g. goserelin acetate, luprolide), inhibitors of growth factors and/or of their corresponding receptors (growth factors such as for example platelet derived growth factor (PDGF), fibroblast growth factor (FGF), vascular endothelial growth factor (VEGF), epidermal growth factor (EGF), insuline-like growth factors (IGF), human epidermal growth factor (HER, e.g. HER2, HER3, HER4) and hepatocyte growth factor (HGF) and/or their corresponding receptors), inhibitors are for example (anti-)growth factor antibodies, (anti-)growth factor receptor antibodies and tyrosine kinase inhibitors, such as for example cetuximab, gefitinib, afatinib, nintedanib, imatinib, lapatinib, bosutinib, bevacizumab, pertuzumab and trastuzumab); antimetabolites (e.g. antifolates such as methotrexate, raltitrexed, pyrimidine analogues such as 5fluorouracil (5fluorineU), ribonucleoside and deoxyribonucleoside analogues, capecitabine and gemcitabine, purine and adenosine analogues such as mercaptopurine, thioguanine, cladribine and pentostatin, cytarabine (ara C), fludarabine); antitumour antibiotics (e.g. anthracyclins such as doxorubicin, doxil (pegylated liposomal doxorubicin hydrochloride, myocet (non-pegylated liposomal doxorubicin), daunorubicin, epirubicin and idarubicin, mitomycin-C, bleomycin, dactinomycin, plicamycin, streptozocin); platinum derivatives (e.g. cisplatin, oxaliplatin, carboplatin); alkylation agents (e.g. estramustin, meclorethamine, melphalan, chlorambucil, busulphan, dacarbazin, cyclophosphamide, ifosfamide, temozolomide, nitrosoureas such as for example carmustin and lomustin, thiotepa); antimitotic agents (e.g. Vinca alkaloids such as for example vinblastine, vindesin, vinorelbin and vincristine; and taxanes such as paclitaxel, docetaxel); angiogenesis inhibitors (e.g. tasquinimod), tubuline inhibitors; DNA synthesis inhibitors, PARP inhibitors, topoisomerase inhibitors (e.g. epipodophyllotoxins such as for example etoposide and etopophos, teniposide, amsacrin, topotecan, irinotecan, mitoxantrone), serine/threonine kinase inhibitors (e.g. PDK 1 inhibitors, Raf inhibitors, A-Raf inhibitors, B-Raf inhibitors, C-Raf inhibitors, mTOR inhibitors, mTORC1/2 inhibitors, PI3K inhibitors, PI3Kα inhibitors, dual mTOR/PI3K inhibitors, STK 33 inhibitors, AKT inhibitors, PLK 1 inhibitors, inhibitors of CDKs, Aurora kinase inhibitors), tyrosine kinase inhibitors (e.g. PTK2/FAK inhibitors), protein protein interaction inhibitors (e.g. IAP activator, Mcl-1, MDM2/MDMX), MEK inhibitors, ERK inhibitors, FLT3 inhibitors, BRD4 inhibitors, IGF-1R inhibitors, TRAILR2 agonists, Bcl-xL inhibitors, Bcl-2 inhibitors, Bcl-2/Bcl-xL inhibitors, ErbB receptor inhibitors, BCR-ABL inhibitors, ABL inhibitors, Src inhibitors, rapamycin analogs (e.g. everolimus, temsirolimus, ridaforolimus, sirolimus), androgen synthesis inhibitors, androgen receptor inhibitors, DNMT inhibitors, HDAC inhibitors, ANG1/2 inhibitors, CYP17 inhibitors, radiopharmaceuticals, proteasome inhibitors, immunotherapeutic agents such as immune checkpont inhibitors (e.g. CTLA4, PD1, PD-L1, PD-L2, LAG3, and TIM3 binding molecules/immunoglobulins, such as e.g. ipilimumab, nivolumab, pembrolizumab), ADCC (antibody-dependent cell-mediated cytotoxicity) enhancers (e.g. anti-CD33 antibodies, anti-CD37 antibodies, anti-CD20 antibodies), T-cell engagers (e.g. bi-specific T-cell engagers (BiTEs^{®}) like e.g. CD3 x BCMA, CD3 x CD33, CD3 x CD19), PSMA x CD3), tumor vaccines and various chemotherapeutic agents such as amifostin, anagrelid, clodronat, filgrastin, interferon, interferon alpha, leucovorin, procarbazine, levamisole, mesna, mitotane, pamidronate and porfimer.

In another aspect not explicitly claimed, the invention relates to a pharmaceutical composition comprising at least one compound of Formula **(I)** - or a pharmaceutically acceptable salt thereof - and optionally at least one pharmaceutically acceptable carrier.

In another aspect not explicitly claimed, the invention relates to a pharmaceutical composition comprising a compound of Formula **(I)** - or a pharmaceutically acceptable salt thereof - and at least one other cytostatic and/or cytotoxic active substance.

In another aspect the invention relates to a pharmaceutical composition comprising a therapeutically effective amount of at least one compound of Formula **(I)** or a pharmaceutically acceptable salt thereof and one or more pharmaceutically acceptable excipients.

Suitable preparations for administering the compounds of the invention will be apparent to those with ordinary skill in the art and include for example tablets, pills, capsules, suppositories, lozenges, troches, solutions - particularly solutions for injection (s.c., i.v., i.m.) and infusion (injectables) - elixirs, syrups, sachets, emulsions, inhalatives or dispersible powders.

Suitable tablets may be obtained, for example, by mixing one or more compounds of Formula **(I)** with known excipients, for example inert diluents, carriers, disintegrants, adjuvants, surfactants, binders and/or lubricants.

The dosage range of the compounds of Formula **(I)** applicable per day is usually from 1 mg to 2000 mg, preferably from 10 to 1000 mg.

The dosage for intravenous use is from 1 mg to 1000 mg with different infusion rates, preferably between 5 mg and 500 mg with different infusion rates.

However, it may sometimes be necessary to depart from the amounts specified, depending on the body weight, age, the route of administration, severity of the disease, the individual response to the drug, the nature of its formulation and the time or interval over which the drug is administered (continuous or intermittent treatment with one or multiple doses per day). Thus, in some cases it may be sufficient to use less than the minimum dose given above, whereas in other cases the upper limit may have to be exceeded. When administering large amounts it may be advisable to divide them up into a number of smaller doses spread over the day.

### GENERAL DEFINITIONS

Terms not specifically defined herein should be given the meanings that would be given to them by one of skill in the art in light of the disclosure and the context. As used in the specification, however, unless specified to the contrary, the following terms have the meaning indicated and the following conventions are adhered to.

In the groups, radicals, or moieties defined below, the number of carbon atoms is often specified preceding the group, for example, C₁₋₆alkyl means an alkyl group or radical having 1 to 6 carbon atoms.

In groups like OH, NH₂, S(O), S(O)₂, CN (cyano), COOH, CF₃ or the like, the skilled artisan can see the radical attachment point(s) to the molecule from the free valences of the group itself.

In case a compound of the present invention is depicted in form of a chemical name and as a formula in case of any discrepancy the formula shall prevail. An asterisk may be used in subformulas to indicate the bond which is connected to the core molecule as defined.

The numeration of the atoms of a substituent starts with the atom which is closest to the core or to the group to which the substituent is attached.

The term **"halogen"** denotes fluorine, chlorine, bromine and/or iodine atoms.

The term **"substituted"** as used herein, means that any one or more hydrogens on the designated atom is replaced with a selection from the indicated group, provided that the designated atom's normal valence is not exceeded, and that the substitution results in a stable compound.

Unless specifically indicated, throughout the specification and appended claims, a given chemical formula or name shall encompass tautomers and all stereo, optical and geometrical isomers (*e*.*g*. enantiomers, diastereomers, *E*/*Z* isomers, *etc*.) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates and hydrates of the free compound or solvates and hydrates of a salt of the compound.

In general, substantially pure stereoisomers can be obtained according to synthetic principles known to a person skilled in the field, *e.g*. by separation of corresponding mixtures, by using stereochemically pure starting materials and/or by stereoselective synthesis. It is known in the art how to prepare optically active forms, such as by resolution of racemic forms or by synthesis, *e.g*. starting from optically active starting materials and/or by using chiral reagents.

Enantiomerically pure compounds of this invention or intermediates may be prepared *via* asymmetric synthesis, for example by preparation and subsequent separation of appropriate diastereomeric compounds or intermediates which can be separated by known methods (*e*.*g*. by chromatographic separation or crystallization) and/or by using chiral reagents, such as chiral starting materials, chiral catalysts or chiral auxiliaries.

Further, it is known to the person skilled in the art how to prepare enantiomerically pure compounds from the corresponding racemic mixtures, such as by chromatographic separation of the corresponding racemic mixtures on chiral stationary phases, or by resolution of a racemic mixture using an appropriate resolving agent, *e.g*. by means of diastereomeric salt formation of the racemic compound with optically active acids or bases, subsequent resolution of the salts and release of the desired compound from the salt, or by derivatization of the corresponding racemic compounds with optically active chiral auxiliary reagents, subsequent diastereomer separation and removal of the chiral auxiliary group, or by kinetic resolution of a racemate (*e*.*g*. by enzymatic resolution); by enantioselective crystallization from a conglomerate of enantiomorphous crystals under suitable conditions, or by (fractional) crystallization from a suitable solvent in the presence of an optically active chiral auxiliary.

The phrase **"pharmaceutically acceptable"** is employed herein to refer to those compounds, materials, compositions, and/or dosage forms which are, within the scope of sound medical judgement, suitable for use in contact with the tissues of human beings without excessive toxicity, irritation, allergic response, or other problem or complication, and commensurate with a reasonable benefit/risk ratio.

As used herein **"pharmaceutically acceptable salts"** refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methylbenzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, *N*-methyl-D-glucamine, potassium, sodium and tris(hydroxymethyl)-aminomethane.

The pharmaceutically acceptable salts of the present invention can be synthesized from the parent compound, which contains a basic or acidic moiety, by conventional chemical methods. Generally, such salts can be prepared by reacting the free acid or base form of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention (*e*.*g*. trifluoro acetate salts), also comprise a part of the invention.

For bivalent groups in which it is crucial to determine which adjacent groups they bind and with which valency, the corresponding binding partners are indicated in brackets where necessary for clarification purposes, as in the following representations: 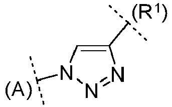 or (R²)-C(O)NH- or (R²)-NHC(O)-;

Groups or substituents are frequently selected from among a number of alternative groups/substituents with a corresponding group designation (*e*.*g*. **R^{a}**, **R^{b}** etc). If such a group is used repeatedly to define a compound according to the invention in different parts of the molecule, it is pointed out that the various uses are to be regarded as totally independent of one another.

The term **"therapeutically effective amount"** as used herein refers to a quantity of substance that is capable of obviating symptoms of illness or of preventing or alleviating these symptoms, or which prolong the survival of a treated patient.

The term **"prodrug"** as used herein refers to (i) an inactive form of a drug that exerts its effects after metabolic processes within the body converting it to a usable or active form, or (ii) a substance that gives rise to a pharmacologically active metabolite, although not itself active (i.e. an inactive precursor).

The terms **"prodrug"** or **"prodrug derivative"** mean a covalently-bonded derivative, carrier or precursor of the parent compound or active drug substance which undergoes at least some biotransformation prior to exhibiting its pharmacological effect(s). Such prodrugs either have metabolically cleavable or otherwise convertible groups and are rapidly transformed in vivo to yield the parent compound, for example, by hydrolysis in blood or by activation via oxidation as in case of thioether groups. Most common prodrugs include esters and amide analogs of the parent compounds. The prodrug is formulated with the objectives of improved chemical stability, improved patient acceptance and compliance, improved bioavailability, prolonged duration of action, improved organ selectivity, improved formulation (e.g., increased hydrosolubility), and/or decreased side effects (e.g., toxicity). In general, prodrugs themselves have weak or no biological activity and are stable under ordinary conditions. Prodrugs can be readily prepared from the parent compounds using methods known in the art, such as those described in A Textbook of Drug Design and Development, Krogsgaard-Larsen and H. Bundgaard (eds.), Gordon & Breach, 1991, particularly Chapter 5: "Design and Applications of Prodrugs"; Design of Prodrugs, H. Bundgaard (ed.), Elsevier, 1985; Prodrugs: Topical and Ocular Drug Delivery, K.B. Sloan (ed.), Marcel Dekker, 1998; Methods in Enzymology, K. Widder et al. (eds.), Vol. 42, Academic Press, 1985, particularly pp. 309-396; Burger's Medicinal Chemistry and Drug Discovery, 5th Ed., M. Wolff (ed.), John Wiley & Sons, 1995, particularly Vol. 1 and pp. 172-178 and pp. 949-982; Pro-Drugs as Novel Delivery Systems, T. Higuchi and V. Stella (eds.), Am. Chem. Soc., 1975; Bioreversible Carriers in Drug Design, E.B. Roche (ed.), Elsevier, 1987.

The term **"pharmaceutically acceptable prodrug"** as used herein means a prodrug of a compound of the invention which is, within the scope of sound medical judgment, suitable for use in contact with the tissues of humans without undue toxicity, irritation, allergic response, and the like, commensurate with a reasonable benefit/risk ratio, and effective for their intended use, as well as the zwitter ionic forms, where possible.

The term **"compound selective over EGFR wild type"** as used herein refers to a compound exhibiting higher efficacy on HER2 compared to EGFR, where the efficacy of the compound can be determined in a biological assay such as a BA/F3 proliferation assay, or a tumor cell line proliferation assay as described below.

The term **"sparing EGFR wild type"** or **"EGFR wild type sparing activity"** as used herein refers to the low EGFR wild type efficacy of a compound, which can be determined in a biological assay such as a BA/F3 proliferation assay, or a tumor cell line proliferation assay as described below.

The term **"cancer with HER2 amplification"** as used herein refers to a cancer where the cancer cells exhibit more than 2 gene copies of ERBB2.

The term **"cancer with HER2 overexpression"** as used herein refers to a cancer, where the cells of the cancer express HER2 at levels detectable by immunohistochemistry and/or methods assaying ERBB2 messenger RNA.

The term **"mutant HER2"** or **"HER2 carrying exon 20 mutation"** as used herein refers to the mutant HER2 protein and concordant mutant DNA variant, whereas **"HER2 exon 20 mutant"** as used herein refers to the HER2 exon 20 mutant protein and concordant mutant DNA variant.

The term **"HER2-mutant cancer"** or **"cancer with HER2 mutations"** refers to a cancer where the cancer or tumor cells harbour HER2 mutation(s) including but not limited to the mutations listed in Table 1 and Table 2.

The term **"cancer with HER2 exon 20 mutation"** or **"HER2 exon 20 mutant cancer"** as used herein refers to a cancer where the cancer or tumor cells harbour at least one HER2 exon 20 mutation including but not limited to the mutations listed in Table 1.

*ERBB2* (HER2) exon 20 encodes for a part of the kinase domain and ranges from amino acids 769 to 835. Every mutation, insertion, duplication or deletion within this region is defined as an exon 20 mutation including mutations listed in Table 1. In addition oncogenic HER2 mutations exist outside of exon 20 including mutations listed in Table 2.

**Table 1. ERBB2 (HER2) exon 20 mutations ("p." is referring to the HER2 protein)**

| |
|---|
| p.A772_G773insMMAY |
| p.Y772_A775_dup (YVMA) |
| p.A775_G776insYVMA |
| p.Y772insYVMA |
| p.M774delinsWLV |
| p.A775_G776insSVMA |
| p.A775_G776insVVMA |
| p.A775_G776insYVMS |
| p.A775_G776insC |
| p.A776_delinsVC |
| p.A776_delinsLC |
| p.A776_delinsVV |
| p.A776_delinsAVGC |
| p.A776_delinsIC |
| p.A776_V777delinsCVC |
| p.V777_insE |
| p.G778_P780dup (GSP) |

**Table 2. Alternative HER2 mutations ("p." is referring to the HER2 protein)**

| |
|---|
| p.S310F |
| p.R678Q |
| p.L755S |
| p.S310Y |
| p.V842I |
| p.D769Y |
| p.D769H |
| p.R103Q |
| p.G1056S |
| p.I767M |
| p.L869R |
| p.L869R |
| p.T733I |
| p.T862A |
| p.V697L |
| p.R929W |
| p.D277H |
| p.D277Y |
| p.G660D |

**List of abbreviations**

| | |
|---|---|
| APCI | atmospheric pressure chemical ionization |
| aq. | aqueous |
| Boc | *tert*-butyloxycarbonyl |
| DMF | *N*,*N*-dimethylformamide |
| DMSO | dimethylsulfoxide |
| ES | electrospray |
| ESI | electrospray ionization |
| FBS | Fetal bovine serum |
| h | hour(s) |
| HPLC | high performance liquid chromatography |
| LC | liquid chromatography |
| min | minutes |
| MS | mass spectrometry |
| MSD | mass selective detector |
| RP | reversed phase |
| sat. | saturated |
| *tert* | tertiary |
| THF | tetrahydrofuran |
| TLC | thin layer chromatography |
| t_{Ret.} | retention time |
| UPLC | Ultra performance liquid chromatography |
| UV | ultraviolet |

Features and advantages of the present invention will become apparent from the following detailed examples, which illustrate the principles of the invention by way of example without restricting its scope as defined in the appended claims:

### Preparation of the compounds according to the invention

### General

The compounds according to the present invention and their intermediates may be obtained using methods of synthesis which are known to the one skilled in the art and described in the literature of organic synthesis. Preferably, the compounds are obtained in analogous fashion to the methods of preparation explained more fully hereinafter, in particular as described in the experimental section. In some cases, the order in carrying out the reaction steps may be varied. Variants of the reaction methods that are known to the one skilled in the art but not described in detail here may also be used.

The general processes for preparing the compounds according to the invention will become apparent to the one skilled in the art studying the following schemes. Starting materials may be prepared by methods that are described in the literature or herein, or may be prepared in an analogous or similar manner. Any functional groups in the starting materials or intermediates may be protected using conventional protecting groups. These protecting groups may be cleaved again at a suitable stage within the reaction sequence using methods familiar to the one skilled in the art.

### General reaction scheme and summary of the synthesis route

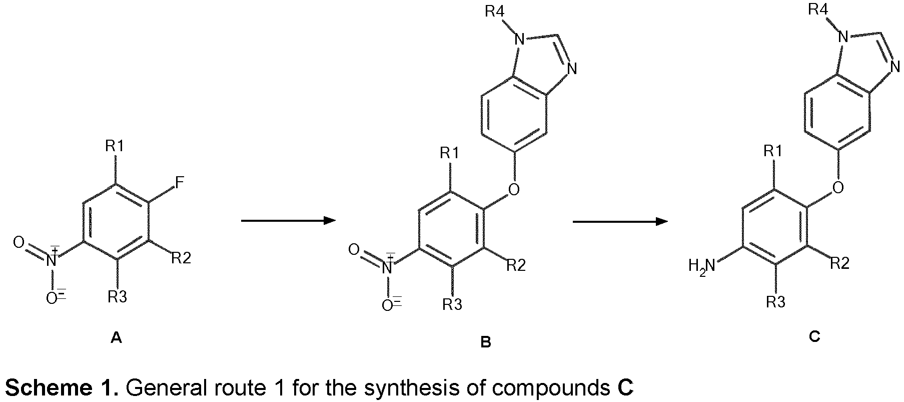

Compounds **C** according to the invention can be synthesized starting from commercially available para-flouronitrobenzenes **(A)** and alcohols, which are reacted in a substitution reaction and subsequent reduction of the nitro-group to yield the corresponding amines C (see e.g. Ishikawa et al., J. Med. Chem. 2011, 54 (23), 8030-8050; McDaniel et al., J. Med. Chem. 2017, 60 (20), 8369-8384).

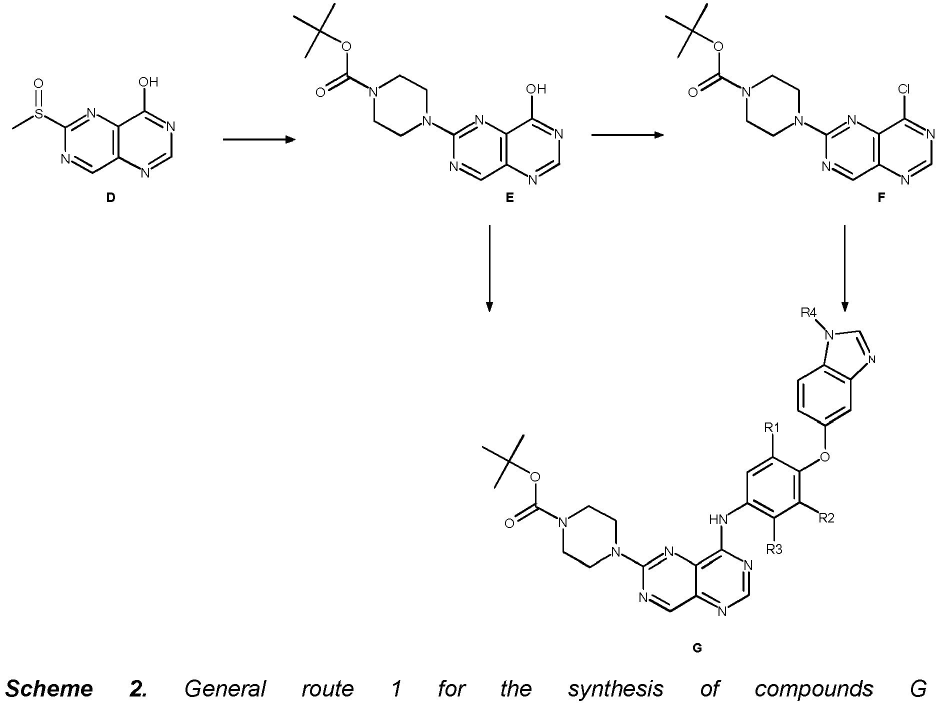

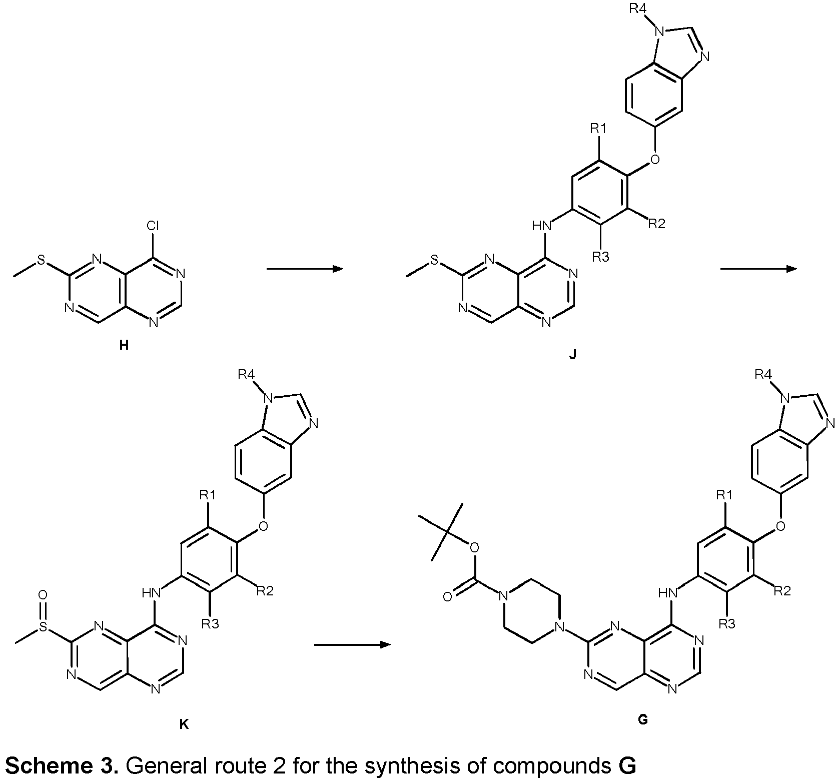

Compounds **G** according to the invention can be synthesized according to General Route 1 from compounds **E** or **F** (see e.g. Wang et al., Bioorg. Med. Chem. Lett. 2016, 26 (11), 2589-2593, Wan et al., Org. Lett. 2006, 8, 11, 2425-2428). Alternatively, compounds G according to the invention can be synthesized according to General Route 2 from compound **H,** which is substituted with the relevant amine (see e.g. Wang et al., Bioorg. Med. Chem. Lett. 2016, 26 (11), 2589-2593), subsequently, the alkylsulfide is oxidized to the sulfoxide or sulfone and substituted with piperazine (see e.g. Del Bello et al., Bioorg. Med. Chem. 2015, 23 (17), 5725-5733).

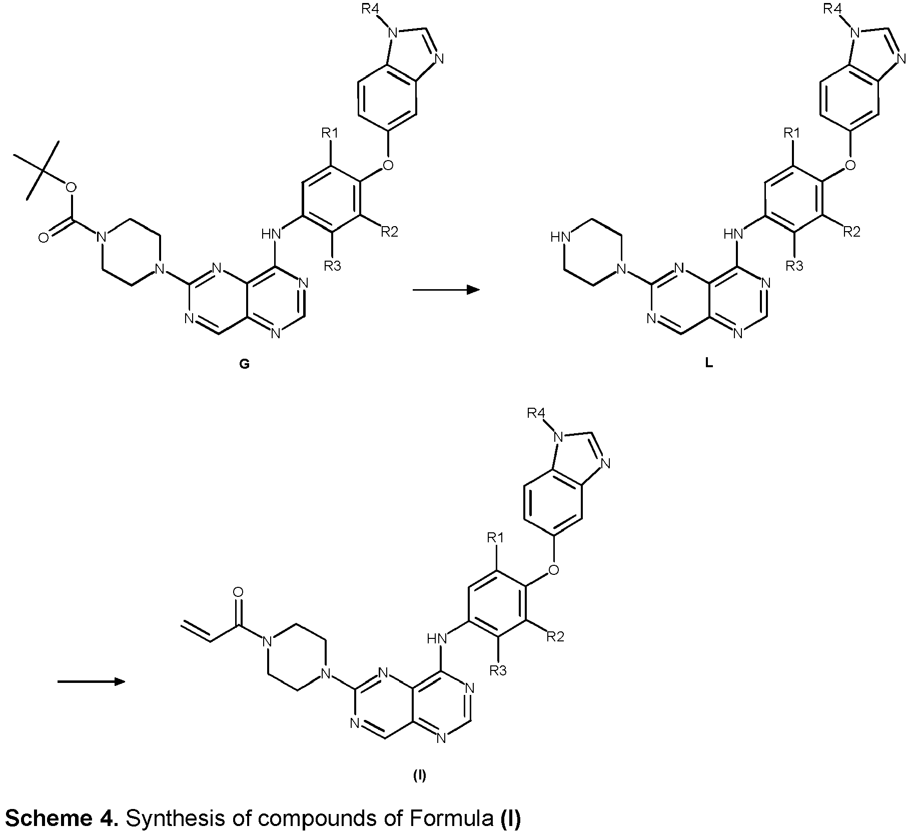

Compounds of Formula **(I)** according to the invention can be synthesized from compounds G by deprotecting the Boc-protected piperazine and subsequent reaction with acryloyl chloride or acryloyl anhydride (see e.g. Zhang et al., Eur. J. Med. Chem. 2019, 178, 417-432.)

Unless stated otherwise, all the reactions are carried out in commercially obtainable apparatus using methods that are commonly used in chemical laboratories. Starting materials that are sensitive to air and/or moisture are stored under protective gas and corresponding reactions and manipulations therewith are carried out under protective gas (nitrogen or argon).

The compounds according to the invention are named in accordance with CAS rules using the software AutoNom (Beilstein) or MarvinSketch (ChemAxon, product version 17.24.3). If a compound is to be represented both by a structural formula and by its nomenclature, in the event of a conflict the structural formula is decisive.

The example compounds of Formula **(I)**, **I-1** to **I-12**, and intermediates are prepared by the methods of synthesis described hereinafter in which the substituents of the general formulae have the meanings given hereinbefore. These methods are intended as an illustration of the invention without restricting its subject matter and the scope of the compounds claimed to these examples. Where the preparation of starting compounds is not described, they are commercially obtainable or their synthesis is described in the prior art or they may be prepared analogously to known prior art compounds or methods described herein, *i.e.* it is within the skills of an organic chemist to synthesize these compounds. Substances described in the literature can be prepared according to the published methods of synthesis.

### Chromatography

Thin layer chromatography is carried out on ready-made silica gel 60 TLC plates on glass (with fluorescence indicator F-254) made by Merck.

**Preparative high pressure chromatography (RP HPLC)** of the example compounds is carried out on Agilent or Gilson systems with columns made by Waters (names: SunFire^{™} Prep C18, OBD^{™} 10 µm, 50 x 150 mm or SunFire^{™} Prep C18 OBD^{™} 5 µm, 30 x 50 mm or XBridge^{™} Prep C18, OBD^{™} 10 µm, 50 x 150 mm or XBridge^{™} Prep C18, OBD^{™} 5 µm, 30 x 150 mm or XBridge^{™} Prep C18, OBD^{™} 5 µm, 30 x 50 mm) and YMC (name: Actus-Triart Prep C18, 5 µm, 30 x 50 mm).

Different gradients of H₂O/acetonitrile are used to elute the compounds, while for Agilent systems 5 % acidic modifier (20 mL HCOOH to 1 L H₂O/acetonitrile (1/1)) is added to the water (acidic conditions). For Gilson systems 0.1 % HCOOH is added to the water.

For the chromatography under basic conditions for Agilent systems H₂O/acetonitrile gradients are used as well, 5 % basic modifier is added to the aqueous eluent (50 g NH₄HCO₃ + 50 mL NH₃ (25 % in H₂O) + H₂O for 1 L aqueous eluent). For Gilson systems the aqueous eluent consists of 5 mL NH₄HCO₃ solution (158 g in 1 L H₂O) and 2 mL NH₃ (28 % in H₂O), replenished to 1 L with H₂O.

**HPLC-MS** columns used were from Waters (XBridge^{™} C18, 2.5 µm, 2.1 × 20 mm or XBridge^{™} C18, 2.5 µm, 2.1 × 30 mm or Aquity UPLC BEH C18, 1.7 µm, 2.1 × 50mm), YMC (Triart C18, 3.0 µm, 2.0 x 30 mm) and Phenomenex (Luna C18, 5.0 µm, 2.0 x 30 mm).

### HPLC-mass spectroscopy/UV-spectrometry

The retention times/MS-ESI⁺ for characterizing the example compounds according to the invention are produced using an HPLC-MS apparatus (high performance liquid chromatography with mass detector). Compounds that elute at the injection peak are given the retention time t_{Ret.} = 0.00.

### Method 1

| | | |
|---|---|---|
| **HPLC** | Agilent 1100/1200 system | |
| **MS** | 1200 Series LC/MSD (MM-ES+APCI + 3000 V, Quadrupol, G6130) | |
| **MSD signal settings column** | Scan pos 150 - 750 | |
| | Waters; Part. No. 186003020; XBridge BEH C18, 3.5 µm), 30 x 2.1 mm column or Waters; Part. No. 186006028; XBridge BEH C18 XP, 2.5 µm, 30 x 2.1 mm column | |
| **eluent** | 5 mM NH₄HCO₃/18 mM NH₃ (pH = 9.2) | |
| | B: acetonitrile (HPLC grade) | |
| **detection signal** | UV 254 nm (bandwidth 8, reference off) | |
| **spectrum** | range: 190 - 400 nm; step: 2 nm | |
| **peak width** | >0.0031 min (0.063 s) (80Hz) | |
| **injection** | 0.5 µL standard injection | |
| **flow** | 1.4 mL/min | |
| **column temperature** | 45 °C | |
| **gradient** | 0.0 - 1.0 min | 15 % → 95 % B |
| | 1.0 - 1.3 min | 95 % B |
| | Stop time: 1.3 min | |

### Method 2

| | | |
|---|---|---|
| **HPLC** | Agilent 1100/1200 system | |
| **MS** | 1200 Series LC/MSD (API-ES +/- 3000 V, Quadrupol, G6140) | |
| **MSD signal settings column** | Scan pos 150 - 750, Scan neg 150 - 750 | |
| | YMC; Part. No. TA12S03-0302WT; Triart C18, 3 µm, 12 nm; 30 x 2.0 mm column | |
| **eluent** | **A**: H₂O + 0.11% formic acid | |
| | B: acetonitrile + 0.1% formic acid (HPLC grade) | |
| **detection signal** | UV 254 nm (bandwidth 10, reference off) | |
| **spectrum** | range: 190 - 400 nm; step: 4 nm | |
| **peak width** | > 0.005 min (0.1 s) | |
| **injection** | 0.5 µL standard injection | |
| **flow** | 1.4 mL/min | |
| **column temperature** | 45°C | |
| **gradient** | 0.0 - 1.0 min | 15 % → 100 % B |
| | 1.0 - 1.1 min | 100 % B |
| | Stop time: 1.23 min | |

### Method 3

| | | |
|---|---|---|
| **HPLC** | Agilent 1100/1200 system | |
| **MS** | 1200 Series LC/MSD (MM-ES+APCI +/- 4000 V, Quadrupol, G6130) | |
| **MSD signal settings column** | Scan pos 150 - 800, Scan neg 150 - 800 | |
| | Waters; Part. No. 186003020; XBridge BEH C18, 3.5 µm, 30 x 2.1 mm column or Waters; Part. No. 186006028; XBridge BEH C18 XP, 2.5µm, 30 x 2.1 mm column | |
| **eluent** | 5 mM NH₄HCO₃/18 mM NH₃ (pH = 9.2) | |
| | B: acetonitrile (HPLC grade) | |
| **detection signal** | UV 254 nm (bandwidth 8, reference off) | |
| **spectrum** | range: 190 - 400 nm; step: 4 nm | |
| **peak width** | >0.0031 min (0.063 s) | |
| **injection** | 0.5 µL standard injection | |
| **flow** | 1.4 mL/min | |
| **column temperature** | 45°C | |
| **gradient** | 0.0 - 1.0 min | 15 % → 95 % B |
| | 1.0 - 1.3 min | 95 % B |
| | Stop time: 1.3 min | |

### Method 4

| | | |
|---|---|---|
| **HPLC** | Agilent 1260 system | |
| **MS** | 1200 Series LC/MSD (API-ES +/- 3000 V, Quadrupol, G6140) | |
| **MSD signal settings** | Scan pos/neg 120 - 900m/z | |
| **column** | Waters, Xbridge C18, 2.5 µm, 2.1x20 mm column | |
| **eluent** | A: 20mM NH₄HCO₃/ NH₃ pH 9 | |
| | B: acetonitrile HPLC grade | |
| **detection signal** | 315 nm (bandwidth 170nm, reference off) | |
| **spectrum** | range: 230 - 400 nm | |
| **peak width** | <0.01 min | |
| **injection** | 5 µL standard injection | |
| **column temperature** | 60°C | |
| **flow** | 1.00 mL/min | |
| **gradient** | 0.00 - 1.50 min | 10 % 4 95% B |
| | 1.50 - 2.00 min | 95 % B |
| | 2.00 - 2.10 min | 95% 4 10 % B |

### Synthesis of compounds C-1 - C-12

### Method 1

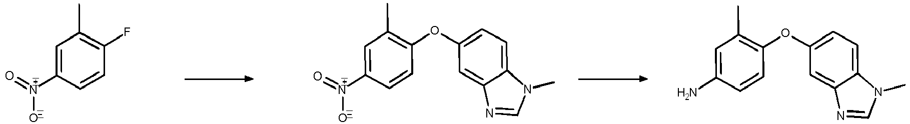

1-Methyl-1*H*-benzo[*d*]imidazol-5-ol (500 mg, 3.38 mmol), 1-fluoro-2-methyl-4-nitrobenzene (680.6 mg, 4.39 mmol) and potassium carbonate (1.16 g, 8.44 mmol) in DMF (5 mL) are stirred at 80°C for 6 h. The reaction mixture is concentrated. The crude product is purified by column chromatography (SiO₂, cyclohexane/ethyl acetate gradient). The appropriate fractions are combined and concentrated to give the intermediate **B-1** (950 mg). This intermediate **B-1** (950 mg, 3.35 mmol) and palladium on charcoal (10%, 100 mg, 0.940 mmol) are suspended in ethanol (10 mL) and stirred under hydrogen pressure (3 bar) at a temperature of 18-25°C overnight. The reaction mixture is filtered and concentrated in vacuo to give the product **C-1** (840 mg).

### Method 2

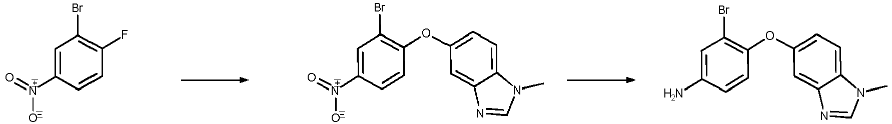

1-Methyl-1*H*-benzo[*d*]imidazol-5-ol (162 mg, 1.09 mmol), 2-bromo-1-fluoro-4-nitrobenzene (240 mg, 1.09 mmol) and potassium carbonate (377 mg, 2.73 mmol) in DMSO (3 mL) are stirred at 80°C for 1 h. The reaction mixture is poured onto water. The intermediate **B-5** is isolated by filtration and washed with copious amounts of water (376 mg). This intermediate **B-5** (376 mg, 1.08 mmol), iron powder (302 mg, 5.40 mmol) and ammonium chloride (600µL sat. aq. solution) are suspended in a mixture of methanol (4 mL) and water (1 mL). The reaction mixture is stirred at 80°C overnight. The reaction mixture is filtered and concentrated in vacuo to give the product **C-5** (340 mg).

Compounds **C-2** and **C-3** (Table 3) are synthesized analogously to the either of the procedures shown above, compounds **C-4, C-6 - C-12** are synthesized analogously to method 2 shown above.

**Table 3**

| **Example Number** | **Structure** | **t_{Ret} [min]** | **[M+H]⁺** | **Method** |
|---|---|---|---|---|
| **C-1** | 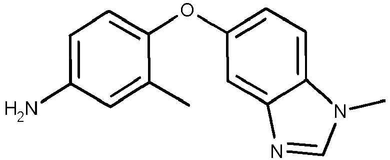 | 0.41 | 254 | 1 |
| **C-2** | 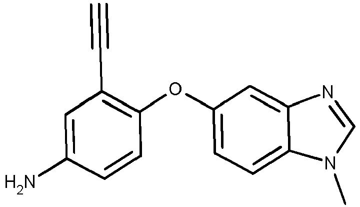 | 0.37 | 264 | 3 |
| **C-3** | 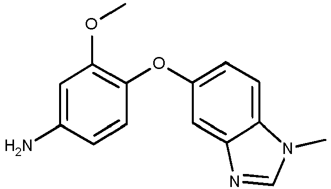 | 0.08 | 270 | 2 |
| **C-4** | 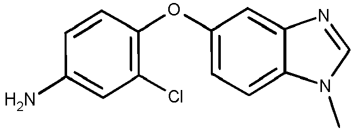 | 0.43 | 274 | 3 |
| **C-5** | 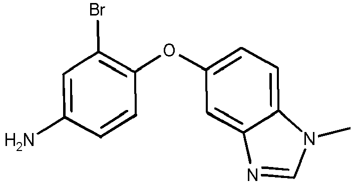 | 0.44 | 318/320 | 1 |
| **C-6** | 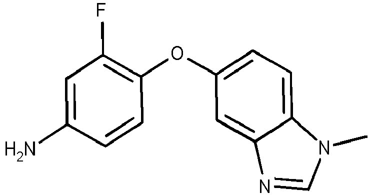 | 0.39 | 258 | 1 |
| **C-7** | 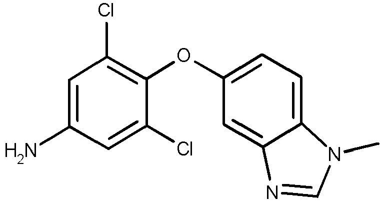 | 0.38 | 308/310 | 2 |
| **C-8** | 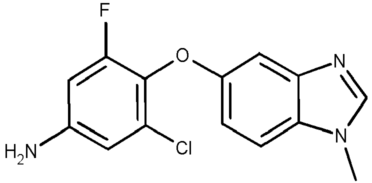 | 0.46 | 292/294 | 3 |
| **C-9** | 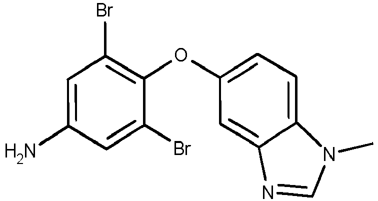 | 0.38 | 398 | 2 |
| **C-10** | 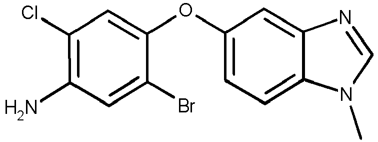 | 0.40 | 352/354 | 2 |
| **C-11** | 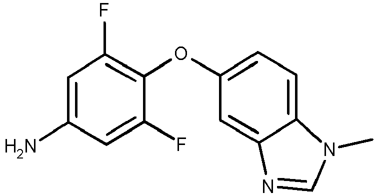 | 0.30 | 276 | 2 |
| **C-12** | 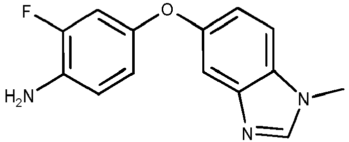 | 0.38 | 258 | 1 |

Compounds **G-1 - G-12** can be prepared according to general route 1 or general route 2 shown below.

### Synthesis of compounds G according to general route 1 (Scheme 2)

### Synthesis of compound E

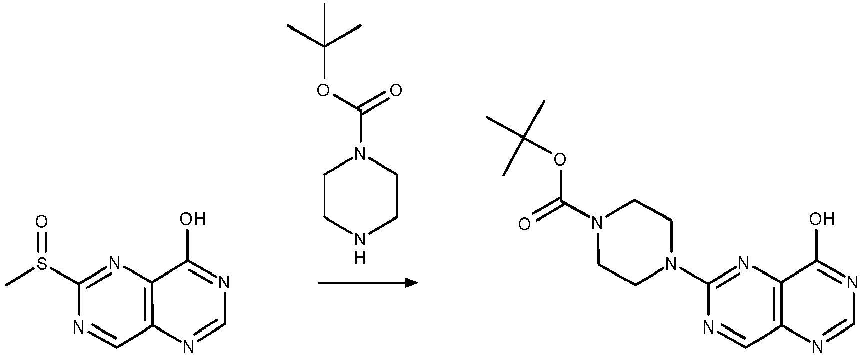

6-Methanesulfinyl[1,3]diazino[5,4-d]pyrimidin-4-ol (10.30 g, 49.0 mmol, prepared according to WO 97/32880) and *tert*-butyl piperazine-1-carboxylate (11.29 g, 186.3 mmol) in dioxane (200 mL) are stirred under reflux for 16 h. The reaction mixture is concentrated under reduced pressure. The crude product is stirred in DMSO (200 mL) at 80°C for 30 min, poured onto ice water (1 L), and stirred for additional 30 min. The solid is collected by filtration, triturated with water, and re-dissolved in dichloromethane/methanol (9/1). The organic layer is washed subsequently with 1 N aq. HCl (200 mL) and brine (200 mL), dried (MgSO₄), filtered and concentrated *in vacuo* to give compound **E** (11.1 g).

**Table 4**

| **Example Number** | **Structure** | **t_{Ret} [min]** | **[M+H]⁺** | **Method** |
|---|---|---|---|---|
| **E** | 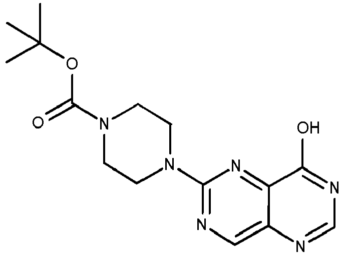 | 0.38 | 333 | 3 |

### Synthesis of compounds G

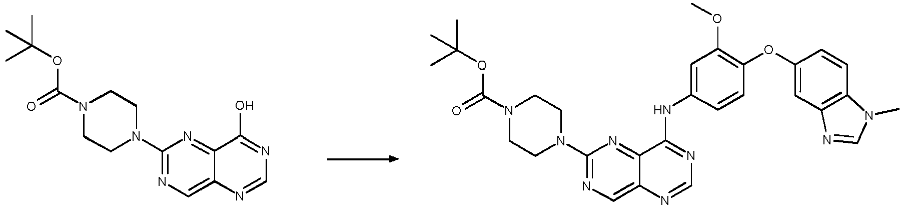

**E** (100 mg, 0.30 mmol), benzotriazol-1-yloxytris(pyrrolidino)phosphonium hexafluorophosphate (230 mg, 0.43 mmol) and 1,8-diazabicyclo[5.4.0]endec-7-ene (69 µL, 0.45 mmol) in dry THF (2 mL) are stirred at a temperature of 18-25°C for 30 min. **C-3** (81 mg, 0.30 mmol) in dry THF (1 mL) is added, and the mixture is stirred at 70°C for 16 h. The mixture is concentrated *in vacuo,* and the crude product G-3 is purified by preparative reversed phase chromatography.

### Synthesis of compound F

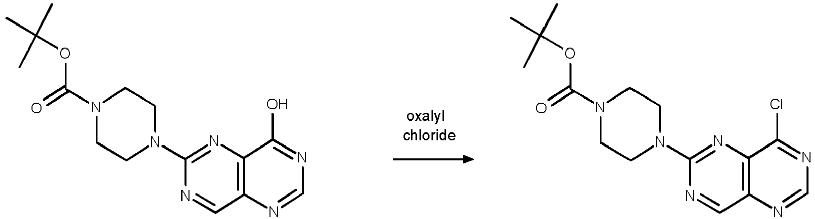

**E** (2.0 g, 6.02 mmol) is suspended in dry dichloromethane (50 mL) and DMF (0.76 mL, 9.79 mmol) is added. Then, oxalyl chloride (1.1 mL, 12 mmol) in dry dichloromethane (10 mL) is added dropwise to the stirred reaction mixture. The reaction mixture is stirred at a temperature of 18-25°C for 1 h, and then concentrated *in vacuo.* The crude product is purified by column chromatography (SiO₂, cyclohexane/ethyl acetate gradient) to yield the product **F** (1.2 g).

**Table 5**

| **Example Number** | **Structure** | **t_{Ret} [min]** | **[M+H]⁺** | **Method** |
|---|---|---|---|---|
| **F** | 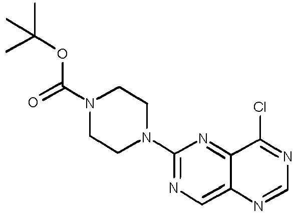 | 1.43 | 351 | 4 |

### Synthesis of compounds G

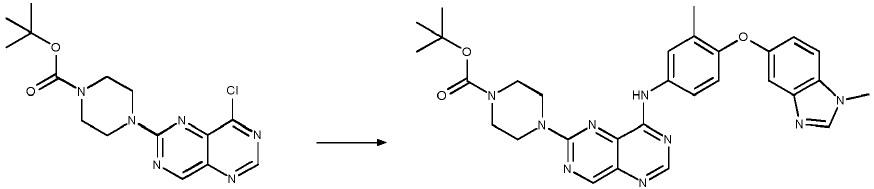

**F** (474 mg, 1.35 mmol) and **C-1** (400 mg, 1.35 mmol) in isopropanol (15 mL) are stirred at 45°C for 4 h. The reaction mixture is concentrated under reduced pressure. The crude product **G-1** (794 mg) is used in the next synthetic step without further purification.

Compounds **G-2, G-4 - G-12** (Table 8) are synthesized analogously to the procedure given above.

### Synthesis of compounds G according to general route 2 (Scheme 3)

Compounds **G-1 - G-12** can also be prepared according to general route 2.

### Synthesis of compounds J

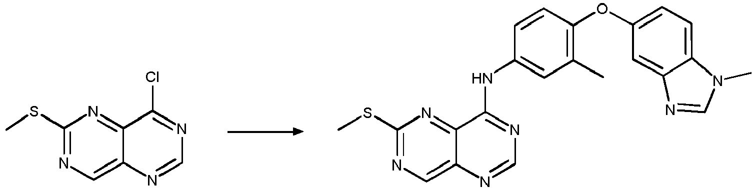

8-Chloro-2-(methylthio)pyrimido[5,4-d]pyrimidine (500 mg, 1.97 mmol) and **C-1** (492 mg, 1.97 mmol) in isopropanol (10 mL) are stirred at 50°C for 3 h. The precipitate is collected by filtration, and the crude product is purified by column chromatography (SiO₂, dichloromethane/methanol gradient) to give the product **J-1** (840 mg).

**Table 6**

| **Example Number** | **Structure** | **t_{Ret} [min]** | **[M+H] ⁺** | **Metho d** |
|---|---|---|---|---|
| **J-1** | 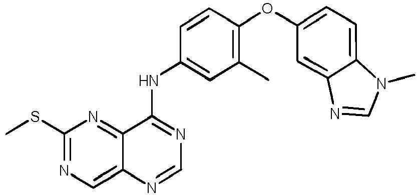 | 0.45 | 430 | 2 |

### Synthesis of compounds K

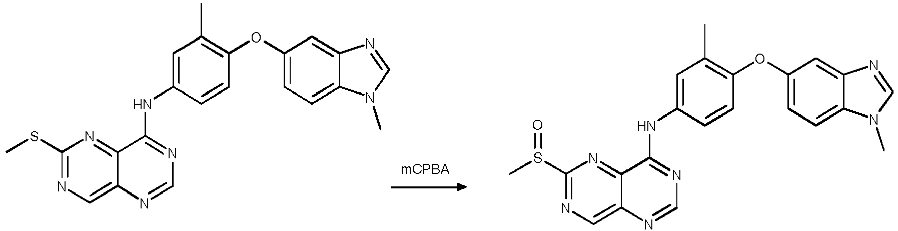

To **J-1** (860 mg, 1.80 mmol) in dichloromethane (30 mL), m-chloroperbenzoic acid (77%, 444 mg, 1.98 mmol) is added at 5°C, and the reaction mixture is stirred at a temperature of 18-25°C for 2 h. Sat. aq. NaHCOs solution (200 mL) is added, and the aqueous layer is extracted with dichloromethane several times. The combined organic layer is washed with water, dried (Mg₂SO₄), filtered, and concentrated *in vacuo.* The crude product **K-1** is used in the next step without further purification (803 mg).

**Table 7**

| **Example Number** | **Structure** | **t_{Ret} [min]** | **[M+H] ⁺** | **Metho d** |
|---|---|---|---|---|
| **K-1** | 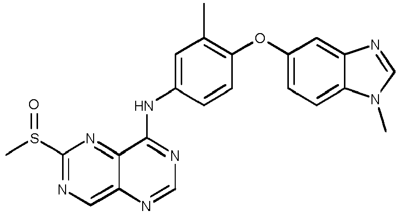 | 0.46 | 446 | 3 |

### Synthesis of compounds G

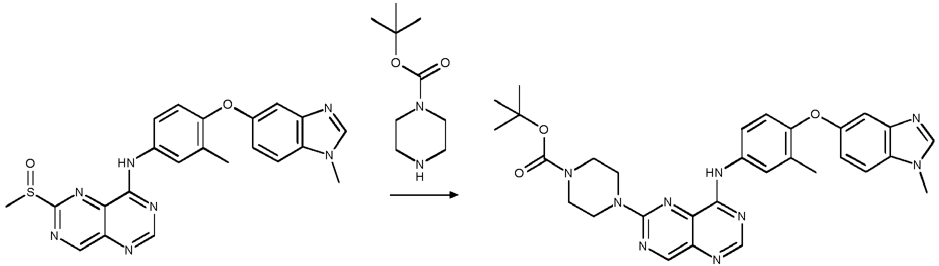

**K-1** (200 mg, 0.43 mmol), tert-butyl piperazine-1-carboxylate (98 mg, 0.51 mmol), and *N,N-*diisopropylethylamine are suspended in dry dioxane (5 mL). The reaction mixtre was stirred at a temperature of 18-25°C for 1 h. The reaction mixture was then heated to 70°C and additional *tert*-butyl piperazine-1-carboxylate (41 mg, 0.21 mmol) was added. The reaction mixture was stirred at 70°C for an additional hour. The reaction mixture is then cooled to a temperature of 18-25°C, concentrated and purified by column chromatography (SiO₂, dichloromethane/methanol gradient) to yield the product **G-1** (175 mg).

**Table 8**

| **Example Number** | **Structure** | **t_{Ret} [min]** | **[M+H]⁺** | **Method** |
|---|---|---|---|---|
| **G-1** | 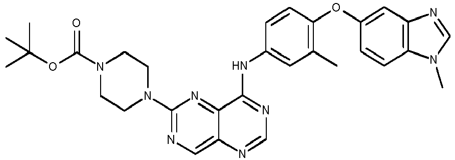 | 0.37 | 568 | 2 |
| **G-2** | 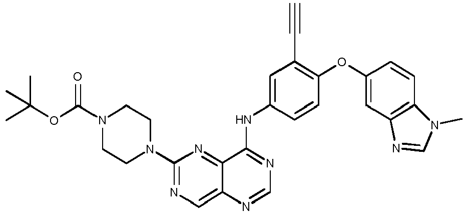 | 0.73 | 578 | 3 |
| **G-3** | 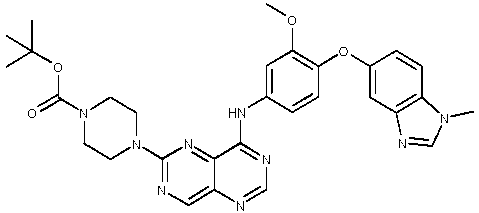 | 0.72 | 584 | 3 |
| **G-4** | 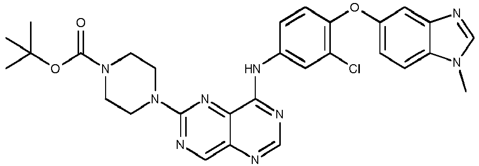 | 0.77 | 588 | 3 |
| **G-5** | 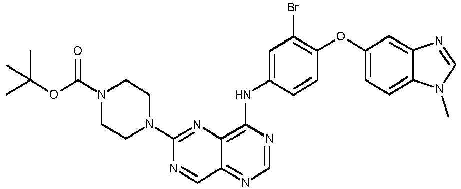 | 0.78 | 632/634 | 1 |
| **G-6** | 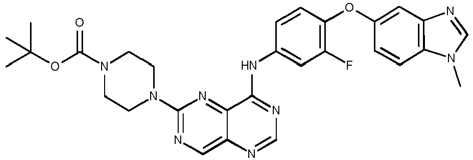 | 0.73 | 572 | 1 |
| **G-7** | 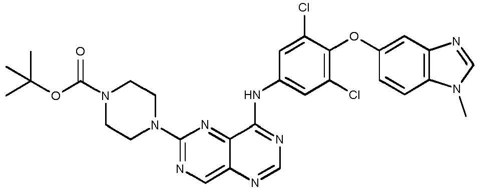 | 0.75 | 622/624 | 2 |
| **G-8** | 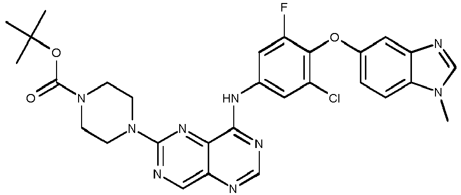 | 0.81 | 606/608 | 3 |
| **G-9** | 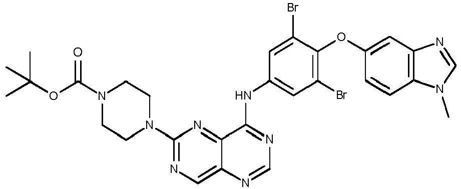 | 0.75 | 712 | 2 |
| **G-10** | 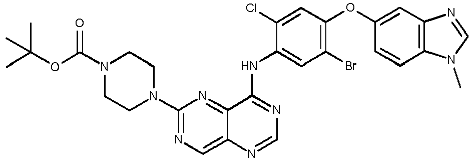 | 0.74 | 668 | 2 |
| **G-11** | 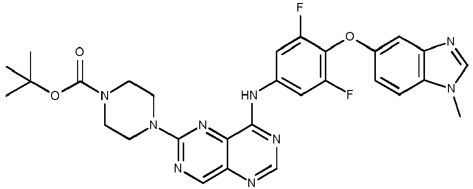 | 0.74 | 590 | 2 |
| **G-12** | 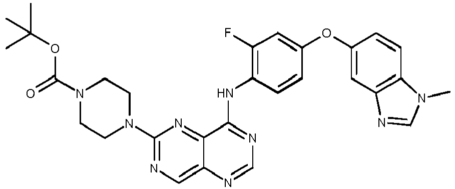 | 0.74 | 572 | 1 |

### Synthesis of compounds L

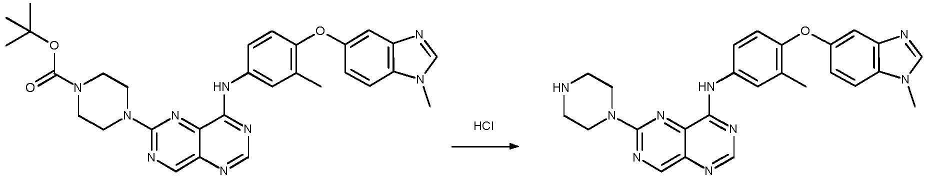

**G-1** (175 mg, 0.31 mmol) is dissolved in dichloromethane (1 mL). Methanol (1 mL) may be added to improve solubility. HCl (4 N in dioxane, 0.5 mL) is added and the reaction mixture is stirred at a temperature of 18-25°C for 2 h at which time HPLC-MS indicated full conversion. The reaction mixture is diluted with methanol, and concentrated under reduced pressure. The crude product is purified by column chromatography (SiO₂, dichloromethane/ammonia in methanol gradient) to yield the product **L-1** (113 mg).

Compounds **L-2** - **L-12** (Table 9) are synthesized analogously to the procedure given above.

**Table 9**

| **Example Number** | **Structure** | **t_{Ret} [min]** | **[M+H]⁺** | **Method** |
|---|---|---|---|---|
| **L-1** | 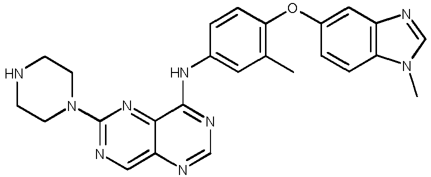 | 0.29 | 468 | 2 |
| **L-2** | 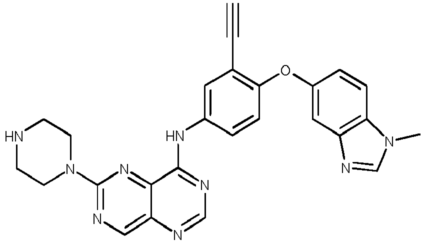 | 0.49 | 478 | 3 |
| **L-3** | 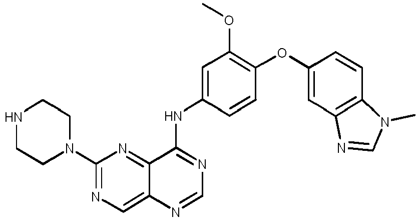 | 0.25 | 484 | 2 |
| **L-4** | 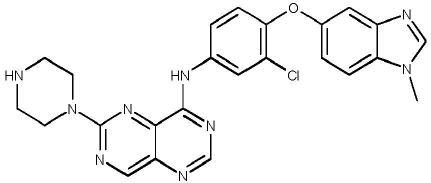 | 0.58 | 542 | 3 |
| **L-5** | 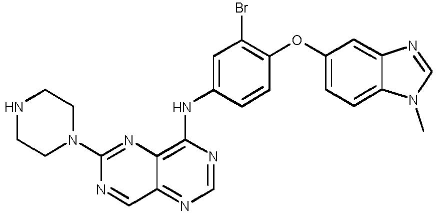 | 0.53 | 532/534 | 1 |
| **L-6** | 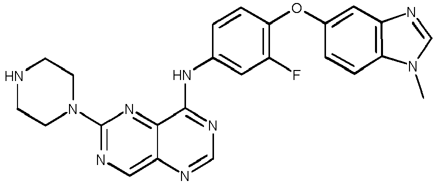 | 0.49 | 472 | 1 |
| **L-7** | 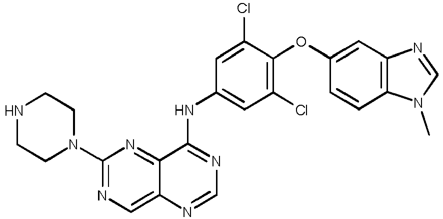 | 0.37 | 522/524 | 2 |
| **L-8** | 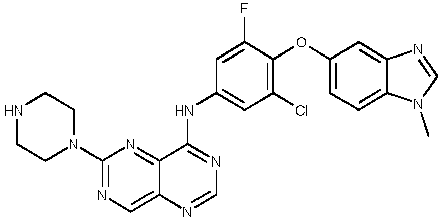 | 0.32 | 506 | 2 |
| **L-9** | 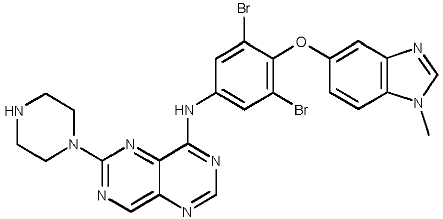 | 0.40 | 612/614 | 2 |
| **L-10** | 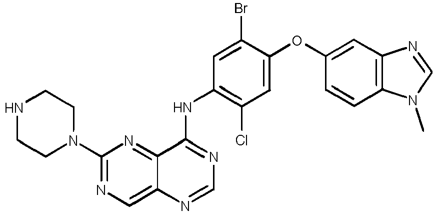 | 0.37 | 566/568 | 2 |
| **L-11** | 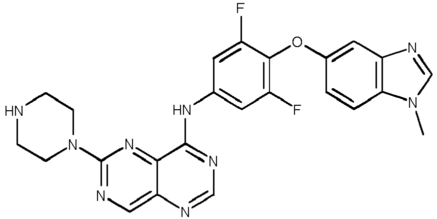 | 0.35 | 490 | 2 |
| **L-12** | 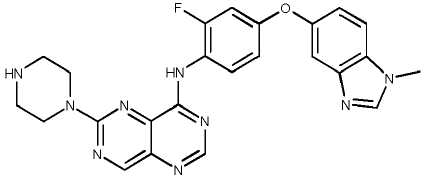 | 0.48 | 472 | 3 |

### Synthesis of compounds I-1 - I-12

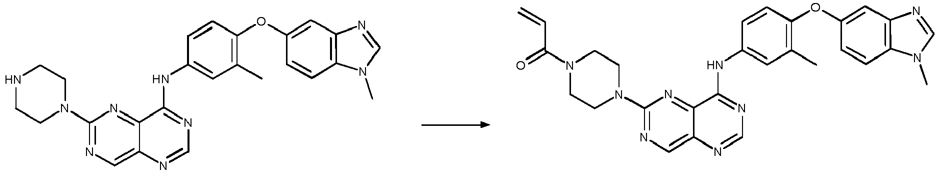

To a solution of **L-1** (200 mg, 0.42 mmol) in dry dichloromethane (7 mL), 4-dimethylaminopyridine (5.1 mg, 0.042 mmol) and trimethylamine (0.18 mL, 1.26 mmol), are added. Then, acrylic anhydride (55 µL, 0.46 mmol) is added dropwise to the stirred reaction mixture. The reaction mixture is stirred at a temperature of 18-25°C for 1 h. The reaction mixture is then diluted with dichloromethane, washed twice with aq. sat. NaHCO₃, dried over Na₂SO₄, filtered, concentrated under reduced pressure and purified by RP-HPLC-MS. The combined product fractions were concentrated to give the product (128 mg).

Alternatively, acryloyl chloride may be used: **L-1** (120 mg, 0.257 mmol) is suspended in dry dichloromethane (5 mL) and triethylamine (214 µL, 2.0 mmol). Acryloyl chloride (32 µL, 0.39 mmol) is added in three portions over 1.5 h. The reaction mixture is stirred at a temperature of 18-25°C for an additional 0.5 h at which time HPLC-MS indicates full conversion. The reaction mixture is diluted with dichloromethane and washed with sat. aq. NaHCOs. The organic layer is dried and concentrated. The crude product is purified by preparative RP-HPLC-MS to yield the product **I-1** (73 mg).

Compounds **I-2** - **I-12** (Table 10) are synthesized analogously to the procedures given above.

**Table 10**

| **Name** | **Structure** | **tRet** | **(M+H)+** | **Method** |
|---|---|---|---|---|
| **I-1** | 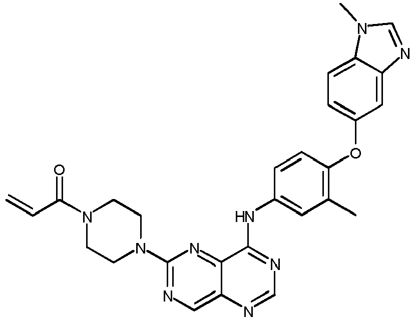 | 1.2 | 522 | 4 |
| **I-2** | 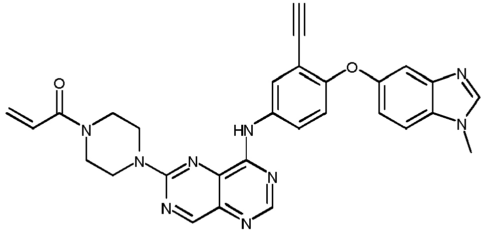 | 1.16 | 532 | 4 |
| **I-3** | 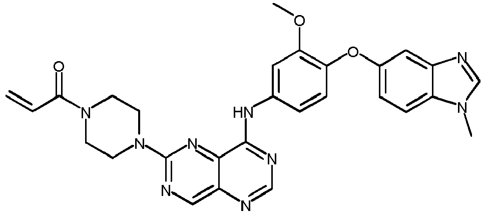 | 1.12 | 538 | 4 |
| **I-4** | 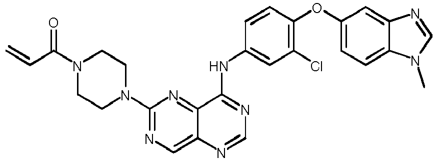 | 1.22 | 542 | 4 |
| **I-5** | 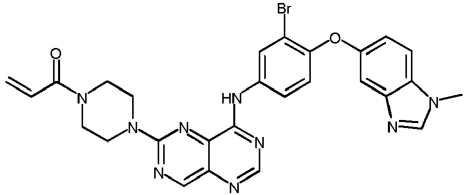 | 1.22 | 586 | 4 |
| **I-6** | 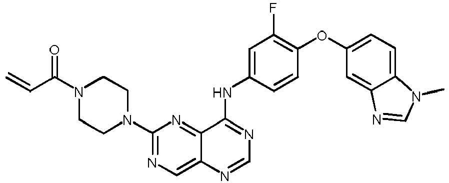 | 1.18 | 526 | 4 |
| **I-7** | 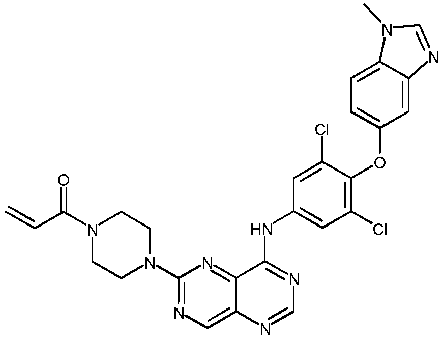 | 1.32 | 576 | 4 |
| **I-8** | 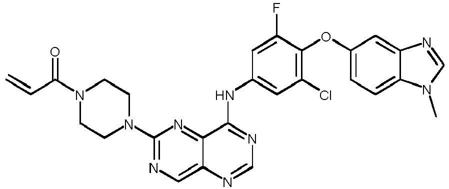 | 1.27 | 560 | 4 |
| **I-9** | 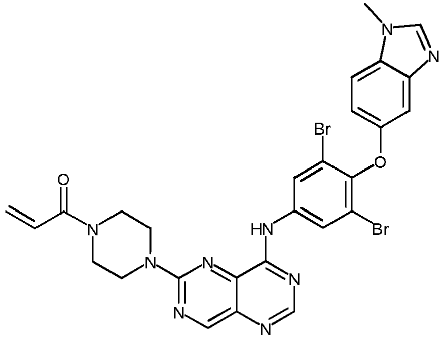 | 1.34 | 664 | 4 |
| **I-10** | 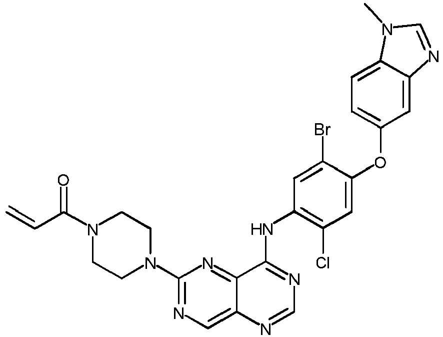 | 1.36 | 620 | 4 |
| **I-11** | 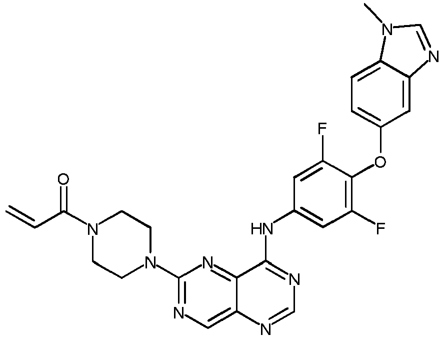 | 1.20 | 544 | 4 |
| **I-12** | 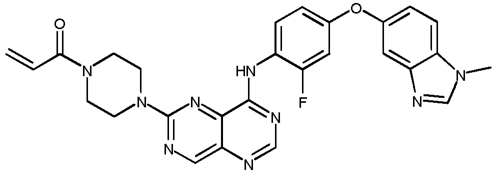 | 1.15 | 526 | 4 |

### Biological assays

### Ba/F3 cell model generation and proliferation assays

Ba/F3 cells were ordered from DSMZ (ACC300) and grown in RPMI-1640 (ATCC 30-2001) + 10 % FBS + 10 ng/ml IL-3 at 37 °C in 5 % CO₂ atmosphere. Plasmids containing HER2 mutants and EGFR WT were obtained from GeneScript. To generate EGFR/HER2-dependent Ba/F3 models, Ba/F3 cells were transduced with retroviruses containing vectors that harbor EGFR WT, HER2 WT or HER2 mutants (YVMA). Platinum-E cells (Cell Biolabs) were used for retrovirus packaging. Retrovirus was added to Ba/F3 cells. To ensure infection, 4 µg/mL polybrene was added and cells were spinfected. Infection efficiency was confirmed by measuring GFP-positive cells using a cell analyzer. Cells with an infection efficiency of 10 % to 20 % were further cultivated and puromycin selection with 1 µg/mL was initiated. As a control, parental Ba/F3 cells were used to assess the selection status. Selection was considered successful when parental Ba/F3 cells cultures died. To evaluate the transforming potential of HER2 mutations, the growth medium was no longer supplemented with IL-3. Ba/F3 cells harboring the empty vector were used as a control. A switch from IL-3 to EGF was performed for Ba/F3 cells with the EGFR WT known for its dependency on EGF ligand. Approximately ten days before conducting the experiments, puromycin was left out. For proliferation assays (data in table 12 and 14), Ba/F3 cells were seeded into 96-well plates at 5 × 10³ cells / 100 µL in growth media. Compounds were added by using a HP D3000 Digital Dispenser. All treatments were performed in technical triplicates. Treated cells were incubated for 72 h at 37 °C with 5 % CO₂. CellTiter-Glo^{®} Luminescent Cell Viability Assay (Promega) was performed and chemoluminescence was measured by using the multilabel Plate Reader VICTOR X4. The raw data were imported into and analyzed with the Boehringer Ingelheim proprietary software MegaLab (curve fitting based on the program PRISM, GraphPad Inc.).

### pEGFR assay

This assay quantifies the phosphorylation of EGFR at Tyr1068 and was used to measure the inhibitory effect of compounds on the transgenic EGFR wild type (WT) protein expressed in HEK cells.

Human HEK cells (ATCC CRL-1573) were grown in Minimum essential Medium Eagle, MEM Eagle EBSS, without L-Glutamine, with non essential amino acids and sodium pyruvate (EMEM Lonza BE12-662F) + 5ml GlutaMax (Gibco 35050-038; L-alanyl-L-glutamine) + 5ml Sodium Pyruvat (Gibco 11360-039; 100 mM) + 10 % FBS at 37 °C in 5 % CO₂ atmosphere and transduced with a retroviral vector encoding EGFR WT.

Transduced cells were selected using puromycin. p-EGFR Tyr1068 was determined using the AlphaScreen Surefire pEGF Receptor (Tyr1068) Assay (PerkinElmer, TGRERS). For the assay, HEK EGFR WT cells were seeded in MEM medium with 10 % FBS. 60 nL compound dilutions were added to each well of Greiner TC 384 plates using the Echo platform. Subsequently, 60.000 cells/well in 60 µL were added. Cells were incubated with compounds for 4 h at 37 °C. Following centrifugation and removal of the medium supernatant, 20 µL of 1.6-fold lysis buffer from TGR/Perkin Elmer kit with protease inhibitors was added. The mixture was incubated at a temperature of 20-25°C with shaking (700 rpm) for 20 min. After centrifugation, 4 µL of the lysate were transferred to Proxiplates. 5 µL of Acceptor Mix (Activation Buffer diluted 1:25 in combined Reaction Buffer 1 and Reaction Buffer 2 (TGRERS Assay Kit, PerkinElmer) plus 1:50 of Protein A Acceptor Beads 6760137, Perkin Elmer) were added to each well. Plates were shaken for 1 min (1400 rpm) and incubated for 2 h at a temperature of 20-25°C in the dark. 3 µL of donor mix (AlphaScreen Streptavidin-coated Donor Beads (6760002, PerkinElmer) 1:50 diluted in Dilution Buffer (TGRERS Assay Kit, PerkinElmer) were added to each well. Plates were shaken for 1 min (1400 rpm) and incubated for 2 h at a temperature of 20-25°C in the dark. Plates were subsequently analyzed using an Envision reader platform. Results were computed in the following way: The ratio of the value of the test compound and the value of the negative control (DMSO) was calculated. IC₅₀ values are computed from these values in the MEGASTAR IC₅₀ application using a 4 parametric logistic model.

This cellular phospho-EGFR (pEGFR) compound dose-response assay quantifies the phosphorylation of EGFR at Tyr1068 in HEK cells expressing EGFR WT. The results of the assay are provided as IC₅₀ values. The higher the pEGFR IC₅₀ values for a given compound, the higher the EGFR WT sparing activity.

### pHER2 (ERBB2) YVMA assay

This assay quantifies the phosphorylation of HER2 YVMA at Tyr1221/1222 and was used to measure the inhibitory effect of compounds on the transgenic HER2 YVMA protein expressed in HEK cells using a Doxycycline inducible expression system.

Human HEK cells were grown in Minimum essential Medium Eagle, MEM Eagle EBSS, without L-Glutamine, with non essential amino acids and sodium pyruvate (EMEM Lonza BE12-662F) + 5ml GlutaMax (Gibco 35050-038; L-alanyl-L-glutamine) + 5ml Sodium Pyruvat (Gibco 11360-039; 100 mM) + 10 % FBS at 37 °C in 5 % CO₂ atmosphere and transduced with a retroviral vector encoding HER2 YVMA. Transduced cells were selected using puromycin. p-HER2 Tyr1221/1222 was determined using the AlphaScreen Surefire ErbB2 (Tyr1221/1222) Assay (PerkinElmer, TGREB2S). For the assay, HEK HER2 YVMA cells were seeded in MEM medium with 10 % FBS. 4 hours prior to compound addition, HER2 YVMA expression was induced using 1 µg/ml Doxycycline. 60 nL compound dilutions were added to each well of Greiner TC 384 plates using the Echo platform. Subsequently, 60.000 cells/well in 60 µL were added. Cells were incubated with compounds for 4 h at 37 °C. Following centrifugation and removal of the medium supernatant, 20 µL of 1.6-fold lysis buffer from TGR/Perkin Elmer kit with protease inhibitors was added. The mixture was incubated at a temperature of 20-25°C with shaking (700 rpm) for 20 min. After centrifugation, 4 µL of the lysate were transferred to Proxiplates. 5 µL of Acceptor Mix (Activation Buffer diluted 1:25 in combined Reaction Buffer 1 and Reaction Buffer 2 (TGREB2S Assay Kit, PerkinElmer) plus 1:50 of Protein A Acceptor Beads 6760137, PerkinElmer) were added to each well. Plates were shaken for 1 min (1400 rpm) and incubated for 2 h at a temperature of 20-25°C in the dark. 3 µL of donor mix (AlphaScreen Streptavidin-coated Donor Beads (6760002, PerkinElmer) 1:50 diluted in Dilution Buffer (TGRERS Assay Kit, PerkinElmer) were added to each well. Plates were shaken for 1 min (1400 rpm) and incubated for 2 h at a temperature of 20-25°C in the dark. Plates were subsequently analyzed using an Envision reader platform. Results were computed in the following way: The ratio of the value of the test compound and the value of the negative control (DMSO) was calculated. IC₅₀ values are computed from these values in the MEGASTAR IC₅₀ application using a 4 parametric logistic model.

This cellular phospho-HER2 YVMA (pHER2 YVMA) compound dose-response assay quantifies the phosphorylation of HER2 YVMA at Tyr1221/1222 in HEK cells expressing HER2 YVMA. The results of the assay are provided as IC₅₀ values. The lower the reported pHER2 YVMA IC₅₀ values for a given compound, the stronger the inhibitory effect of a compound on HER2 YVMA kinase activity.

### HER2 YVMA tumor cell line model generation and proliferation assays

HER2 WT-dependent NCI-H2170 cells were ordered from (ATCC, CRL-5928) and grown in RPMI-1640 (Gibco # A10491) ATCC-Formulation + 10 % FBS at 37 °C in 5 % CO₂ atmosphere. Homology-directed genome engineering was employed to insert a 12 nucleotide sequence encoding YVMA into Exon 20 of the genomic HER2 locus in NCI-H2170 cells. This resulted in the change from HER2 WT to the HER2 YVMA variant representing HER2 p.A775_G776insYVMA. The DNA template containing the HER2 exon 20 YVMA insertion variant was obtained from GenScript. PCR followed by Sanger sequencing was used to confirm the presence of the 12-nucleotide insertion in HER2 Exon 20 resulting in the YVMA amino acid duplication.

For proliferation assays, NCI-H2170 (HER2 wild type), NCI-H2170 HER2 YVMA and EGFR WT dependent A431 cells were used. NCI-H2170 HER2 YVMA or NCI-H2170 cells were seeded into 96-well plates at 750 cells / 60 µL in growth media (RPMI ATCC+ 10% FBS, + Penicillin/Streptomycin). A431 cells (ATCC CRL-1555) (DMEM (Sigma #D6429) + 5ml Sodium Pyruvat Gibco 11360-039) were plated at a density of 5000 cells per well (200µl) in a 96-well plate. Compounds were added by using a HP D3000 Digital Dispenser one day after plating the cells. All treatments were performed in technical triplicates. Treated cells were incubated for 72 h at 37 °C with 5 % CO₂. CellTiter-Glo^{®} Luminescent Cell Viability Assay (Promega) was performed and chemoluminescence was measured by using the multilabel Plate Reader VICTOR X4. The raw data were imported into and analyzed with the Boehringer Ingelheim proprietary software MegaLab (curve fitting based on the program PRISM, GraphPad Inc.).

**Table 11. Biomarker Assay**

| **Name** | **IC50 HEK pHER2 YVMA [nM]** | **IC50 HEK pEGFR [nM]** |
|---|---|---|
| **I-1** | 10 | 265 |
| **I-2** | 10 | 380 |
| **I-3** | 17 | 1170 |
| **I-4** | 10 | 221 |
| **I-5** | 15 | 232 |
| **I-6** | 19 | 1110 |
| **I-7** | 30 | 996 |
| **I-8** | 22 | 355 |
| **I-9** | 34 | 485 |
| **I-10** | 80 | 1770 |
| **I-11** | 25 | 1170 |
| **I-12** | 27 | 873 |

**Table 12. Tumour Cell Proliferation Assay**

| **Name** | **GI50 NCl H-2170 HER2 wt amp. [nM]** | **GI50 NCl H-2170 HER2 YVMA [nM]** | **GI50 A431 EGFR wt amp. [nM]** | **GI50 BAF3 HER2 WT [nM]** | **GI50 BAF3 HER2 YVMA [nM]** | **GI50 BAF3 EGF dep. [nM]** |
|---|---|---|---|---|---|---|
| **I-1** | 16 | 82 | > 5000 | 1 | 18 | 2400 |
| **I-2** | 9 | 50 | 4660 | 1 | 9 | 1380 |
| **I-3** | 18 | 74 | > 5000 | 1 | 12 | 2800 |
| **I-4** | 11 | 81 | >5000 | 2 | 17 | 904 |
| **I-5** | 16 | 76 | 2880 | 1 | 20 | 572 |
| **I-6** | 26 | 146 | 2640 | 3 | 27 | 1840 |
| **I-7** | 25 | 118 | > 5000 | 6 | 37 | 1110 |
| **I-8** | 21 | 172 | 3930 | 3 | 31 | 1040 |
| **I-9** | 46 | 203 | 2510 | 5 | 23 | 1120 |
| **I-10** | 52 | 216 | > 5000 | 8 | 64 | 1630 |
| **I-11** | 47 | 339 | > 5000 | 3 | 40 | 644 |
| **I-12** | 45 | 167 | 4810 | 7 | 48 | 1180 |

**Table 13. Biomarker Assay**

| **Name** | **Structure** | **IC50 HEK pHER2 YVMA [nM]** | **IC50 HEK pEGFR [nM]** |
|---|---|---|---|
| allitinib | 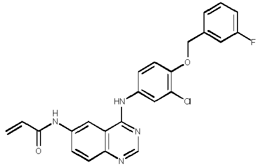 | 3 | 1 |
| ibrutinib | 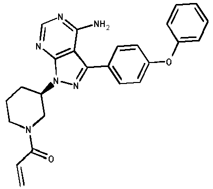 | 26 | 21 |
| neratinib | 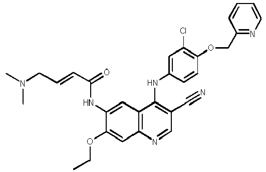 | 1 | 4 |
| poziotinib | 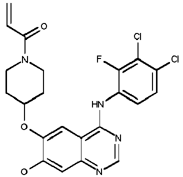 | 5 | 1 |
| WO 2019/046775 Comp.2 | 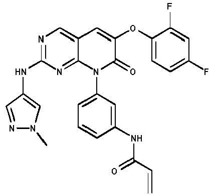 | 11 | 7 |
| tucatinib | 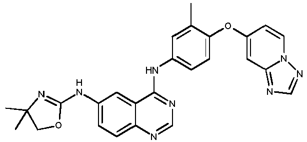 | 4 | 1410 |
| allitinib | 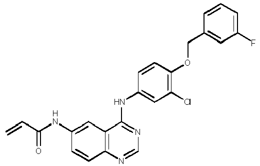 | 3 | 1 |
| WO 2007/059257 Ex.185 | 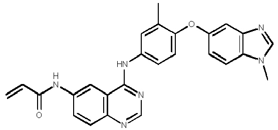 | 33 | 317 |

**Table 14. Tumour Cell Proliferation Assay**

| **Name** | **GI50 NCI H-2170 HER2 wt amp. [nM]** | **GI50 NCI H-2170 HER2 YVMA [nM]** | **GI50 A431 EGFR wt amp. [nM]** | **GI50 BAF3 HER2 WT [nM]** | **GI50 BAF3 HER2 YVMA [nM]** | **GI50 BAF3 EGF dep. [nM]** |
|---|---|---|---|---|---|---|
| allitinib | 36 | 203 | 403 | | 17 | 31 |
| ibrutinib | 16 | 132 | 199 | | 44 | 70 |
| neratinib | 3 | 37 | 55 | 1 | 5 | 14 |
| poziotinib | 1 | 5 | 1 | 1 | 2 | 1 |
| WO 2019/046775 Comp.2 | 22 | 34 | 16 | 3 | 14 | 13 |
| tucatinib | 58 | 798 | 4220 | 10 | 338 | 2970 |
| WO 2007/059257 Ex.185 | 108 | 926 | >5000 | | 60 | 1190 |

### Example of pharmaceutical formulation

| **Ingredient** | **Amount** |
|---|---|
| Active substance | 100 mg |
| lactose | 140 mg |
| corn starch | 240 mg |
| polyvinylpyrrolidone | 15 mg |
| magnesium stearate | 5 mg |

The active substance is grounded and mixed together with lactose and some of the corn starch. The mixture is sieved, then wet granulated with polyvinylpyrrolidone solution. The granules, the remaining corn starch and the magnesium stearate are mixed together. The mixture is compressed to produce tablets of suitable shape and size.

## Claims

1. A compound of Formula **(I)** 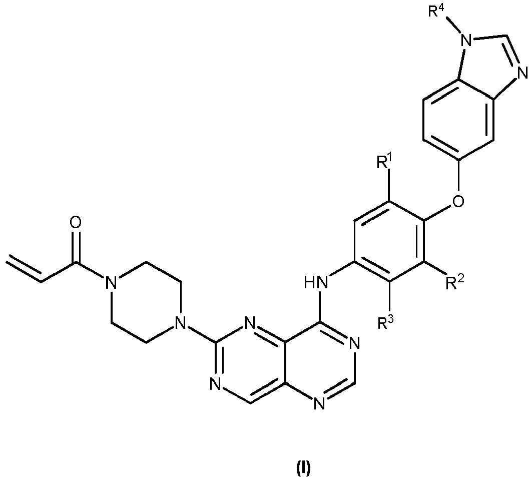 wherein
**R¹** is selected from the group consisting of hydrogen, -CH₃, -CCH, -OCH₃ and halogen
**R²** is hydrogen or halogen
**R³** is hydrogen or halogen
**R⁴** is hydrogen or -CH₃
and at least one of **R¹**, **R²** and **R³** is not hydrogen
or a salt thereof.

2. A compound according to claim 1, wherein
**R¹** is selected from the group consisting of -CH₃, -CCH, -OCH₃ and halogen
or a salt thereof.

3. A compound according to claim 1, wherein
**R¹** is -CH₃
or a salt thereof.

4. A compound according to claim 1, wherein
**R¹** is chlorine
or a salt thereof.

5. A compound according to claim 1, wherein
**R¹** is fluorine
or a salt thereof.

6. A compound according to claim 1, wherein
**R²** is hydrogen
or a salt thereof.

7. A compound according to claim 1, wherein
**R²** is selected from the group consisting of chlorine, bromine and fluorine
or a salt thereof.

8. A compound according to claim 1 or 7, wherein
**R³** is chlorine, fluorine or hydrogen
or a salt thereof.

9. A compound according to claim 1 to 8, wherein
**R⁴** is -CH₃
or a salt thereof.

10. A compound according to claim 1 or a pharmaceutically acceptable salt thereof, selected from the group consisting of examples **I-01** to **I-12:**
| | |
|---|---|
| **I-01** | 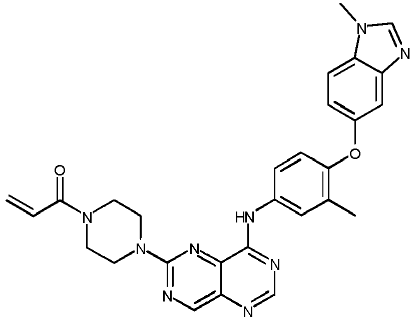 |
| **I-02** | 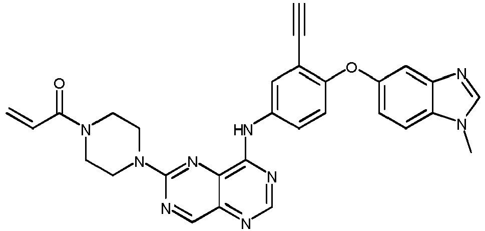 |
| **I-03** | 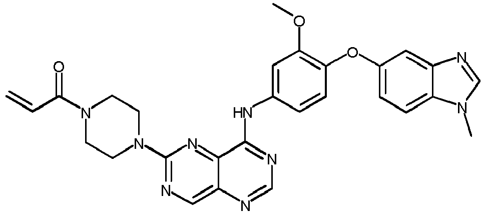 |
| **I-04** | 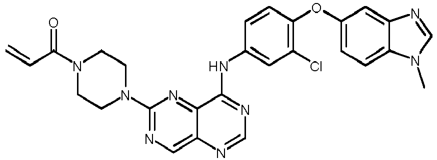 |
| **I-05** | 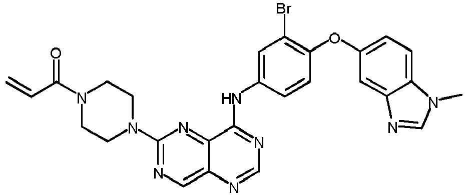 |
| **I-06** | 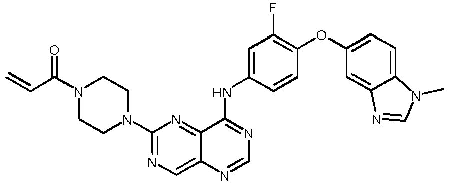 |
| **I-07** | 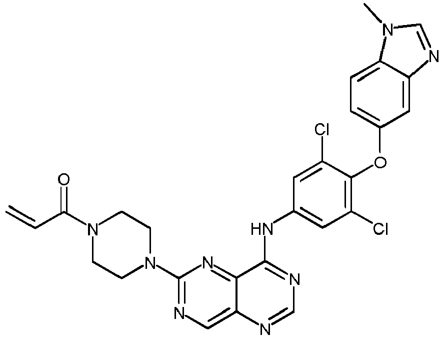 |
| **I-08** | 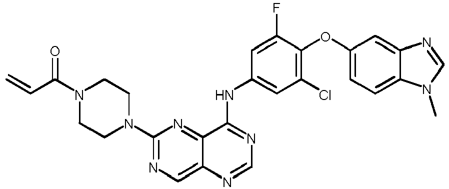 |
| **I-09** | 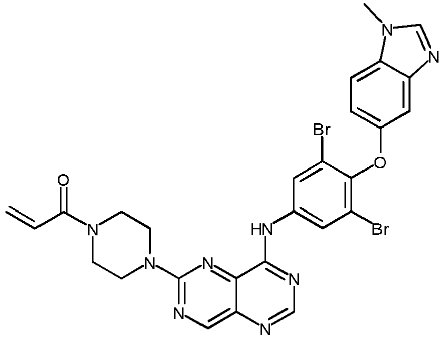 |
| **I-10** | 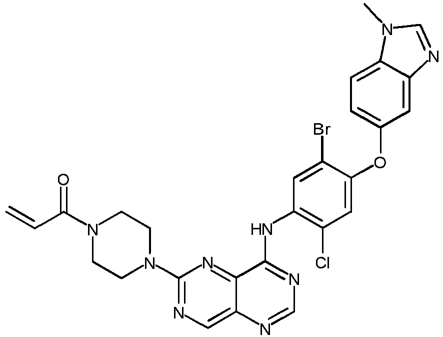 |
| **I-11** | 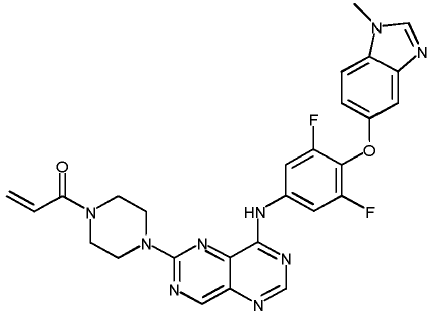 |
| **I-12** | 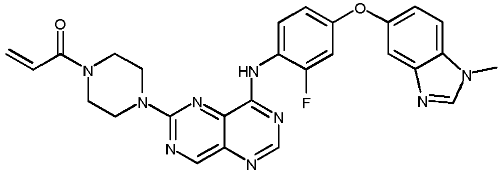 |

11. The compound according to any one of claims 1-3, 6 or 8-10 having the following structure:

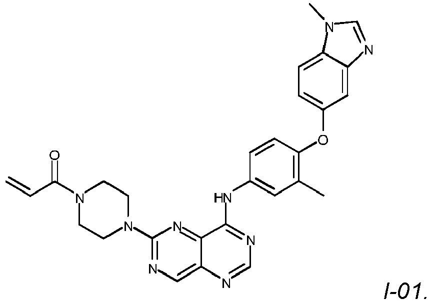

12. A pharmaceutically acceptable salt of the compound according to any one of claims 1-3, 6 or 8-10 having the following structure:

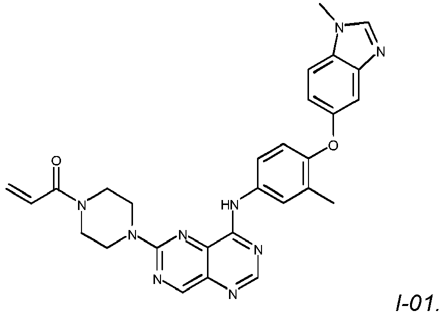

13. A pharmaceutical composition comprising a therapeutically effective amount of at least one compound of Formula **(I)** according to any one of claims 1 to 12 or a pharmaceutically acceptable salt thereof and one or more pharmaceutically acceptable excipients.

14. A compound according to one or more of claims 1 to 12, or a pharmaceutically acceptable salt thereof for use as a medicament.

15. The compound according to one or more of claims 1 to 12 for use in the treatment of brain cancer, breast cancer, biliary cancer, bladder cancer, cervical cancer, colorectal cancer, endometrial cancer, skin cancer, esophagus tumor, head and neck tumor, gastrointestinal cancer, gallbladder tumor, kidney cancer, liver cancer, lung cancer or prostate cancer.

## Patentansprüche

1. Verbindung der Formel (I) 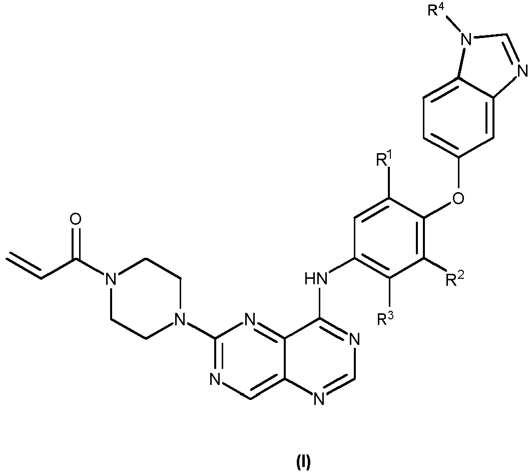 wobei
**R¹** ausgewählt ist aus der Gruppe, bestehend aus Wasserstoff, -CH₃, -CCH, -OCH₃ und Halogen,
**R²** Wasserstoff oder Halogen darstellt,
**R³** Wasserstoff oder Halogen darstellt,
**R⁴** Wasserstoff oder -CH₃ darstellt,
und mindestens eines von **R¹**, **R²** und **R³** ist nicht Wasserstoff,
oder ein Salz hiervon.

2. Verbindung nach Anspruch 1, wobei
**R¹** ausgewählt ist aus der Gruppe, bestehend aus -CH₃, -CCH, -OCH₃ und Halogen,
oder ein Salz hiervon.

3. Verbindung nach Anspruch 1, wobei
**R¹** -CH₃ darstellt,
oder ein Salz hiervon.

4. Verbindung nach Anspruch 1, wobei
**R¹** Chlor darstellt,
oder ein Salz hiervon.

5. Verbindung nach Anspruch 1, wobei
**R¹** Fluor darstellt,
oder ein Salz hiervon.

6. Verbindung nach Anspruch 1, wobei
**R²** Wasserstoff darstellt,
oder ein Salz hiervon.

7. Verbindung nach Anspruch 1, wobei
**R²** ausgewählt ist aus der Gruppe, bestehend aus Chlor, Brom und Fluor,
oder ein Salz hiervon.

8. Verbindung nach Anspruch 1 oder 7, wobei
**R³** Chlor, Fluor oder Wasserstoff darstellt,
oder ein Salz hiervon.

9. Verbindung nach Anspruch 1 bis 8, wobei
**R⁴** -CH₃ darstellt,
oder ein Salz hiervon.

10. Verbindung nach Anspruch 1 oder ein pharmazeutisch akzeptables bzw. annehmbares Salz hiervon, ausgewählt aus der Gruppe, bestehend aus den Beispielen **I-01** bis **I-12:**
| | |
|---|---|
| **I-01** | 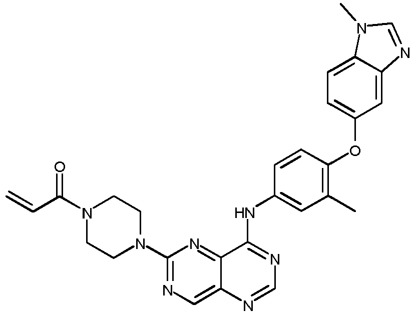 |
| **I-02** | 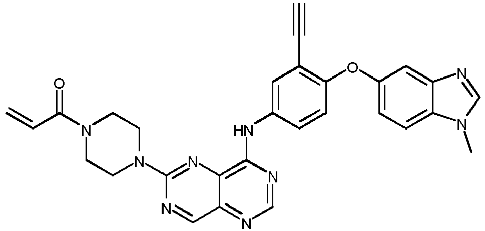 |
| **I-03** | 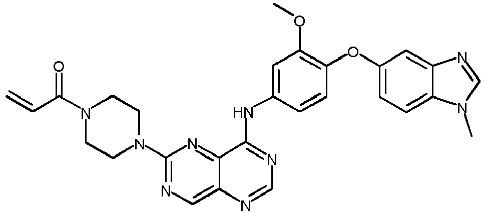 |
| **I-04** | 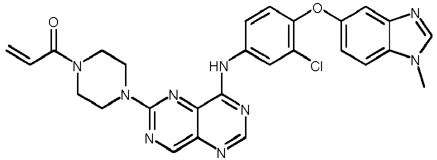 |
| **I-05** | 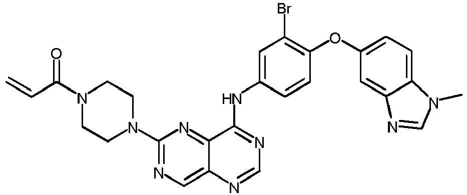 |
| **I-06** | 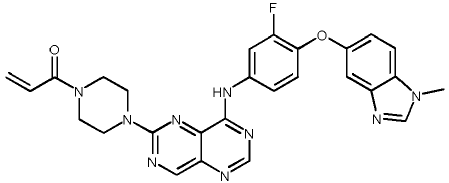 |
| **I-07** | 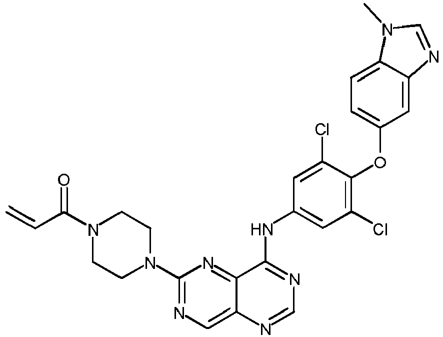 |
| **I-08** | 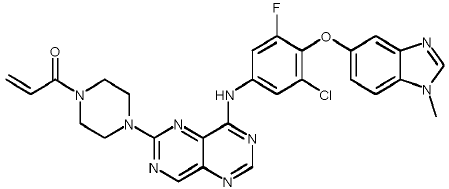 |
| **I-09** | 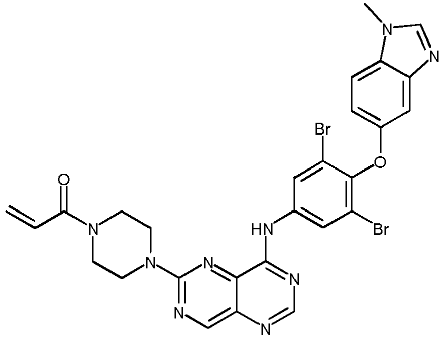 |
| **I-10** | 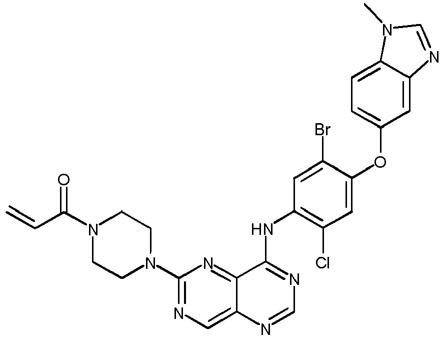 |
| **I-11** | 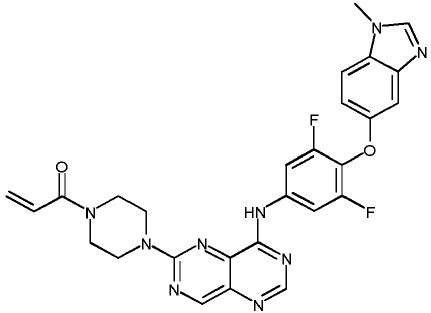 |
| **I-12** | 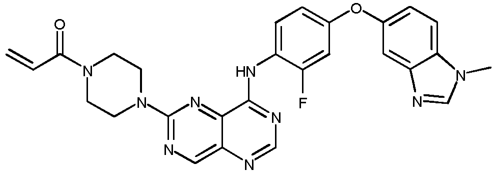 |

11. Verbindung nach irgendeinem der Ansprüche 1-3, 6 oder 8-10 mit der folgenden Struktur:

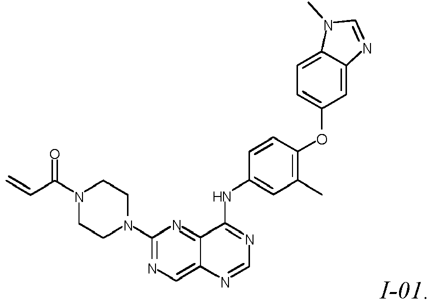

12. Pharmazeutisch akzeptables bzw. annehmbares Salz der Verbindung nach irgendeinem der Ansprüche 1-3, 6 oder 8-10 mit der folgenden Struktur:

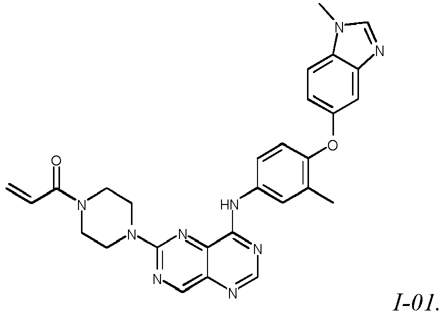

13. Pharmazeutische Zusammensetzung, umfassend eine therapeutisch wirksame Menge von mindestens einer Verbindung der Formel (I) nach irgendeinem der Ansprüche 1 bis 12 oder ein pharmazeutisch akzeptables bzw. annehmbares Salz hiervon und ein oder mehrere pharmazeutisch akzeptable bzw. annehmbare Hilfsstoffe.

14. Verbindung nach einem oder mehreren der Ansprüche 1 bis 12 oder ein pharmazeutisch akzeptables bzw. annehmbares Salz hiervon zur Verwendung als Medikament.

15. Verbindung nach einem oder mehreren der Ansprüche 1 bis 12 zur Verwendung in der Behandlung von Gehirntumor, Brustkrebs, Gallengangkrebs, Blasenkrebs, Gebärmutterhalskrebs, Kolorektalkrebs, Endometriumkrebs, Hautkrebs, Speiseröhrentumor, Kopf-Hals-Tumor, Magen-Darm-Krebs, Gallenblasentumor, Nierenkrebs, Leberkrebs, Lungenkrebs oder Prostatakrebs.

## Revendications

1. Composé de formule **(I)** 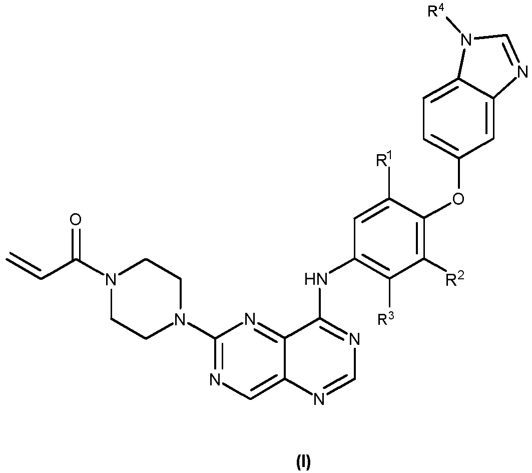 dans laquelle
**R¹** est sélectionné dans le groupe consistant en un hydrogène, -CH₃, -CCH, -OCH₃ et un halogène
**R²** est un hydrogène ou un halogène
**R³** est un hydrogène ou un halogène
**R⁴** est un hydrogène ou -CH₃
et au moins un parmi **R¹**, **R²** et **R³** n'est pas un hydrogène ou un sel de celui-ci.

2. Composé selon la revendication 1, dans lequel
**R¹** est sélectionné dans le groupe consistant en -CH₃, - CCH, -OCH₃ et un halogène
ou un sel de celui-ci.

3. Composé selon la revendication 1, dans lequel
**R¹** est -CH₃
ou un sel de celui-ci.

4. Composé selon la revendication 1, dans lequel
**R¹** est un chlore
ou un sel de celui-ci.

5. Composé selon la revendication 1, dans lequel
**R¹** est un fluor
ou un sel de celui-ci.

6. Composé selon la revendication 1, dans lequel
**R²** est un hydrogène
ou un sel de celui-ci.

7. Composé selon la revendication 1, dans lequel
**R²** est sélectionné dans le groupe consistant en un chlore, un brome et un fluor
ou un sel de celui-ci.

8. Composé selon la revendication 1 ou 7, dans lequel
**R³** est un chlore, un fluor ou un hydrogène
ou un sel de celui-ci.

9. Composé selon les revendications 1 à 8, dans lequel
**R⁴** est -CH₃
ou un sel de celui-ci.

10. Composé selon la revendication 1 ou sel pharmaceutiquement acceptable de celui-ci, sélectionné dans le groupe consistant en les exemples **I-01** à **I-12** :
| | |
|---|---|
| I-01 | 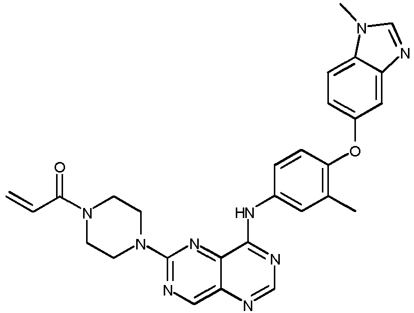 |
| I-02 | 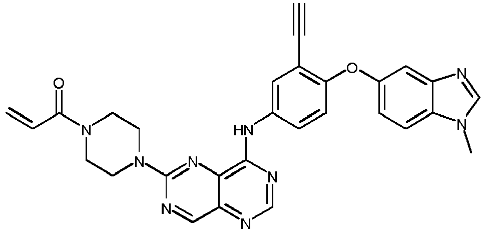 |
| I-03 | 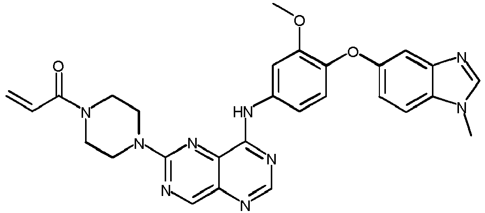 |
| I-04 | 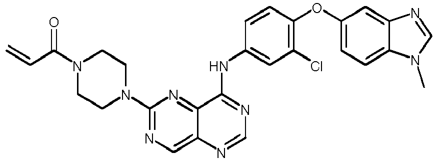 |
| I-05 | 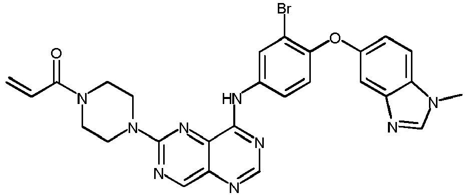 |
| I-06 | 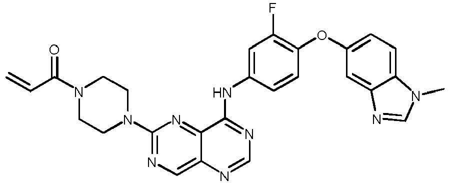 |
| I-07 | 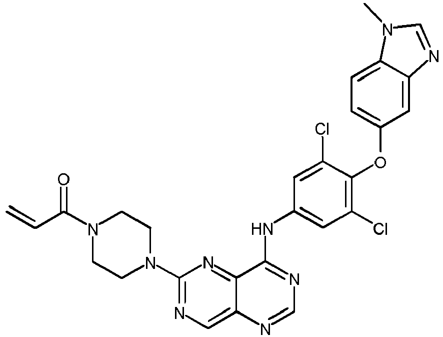 |
| I-08 | 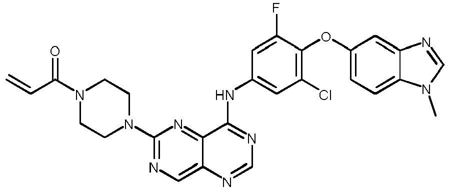 |
| I-09 | 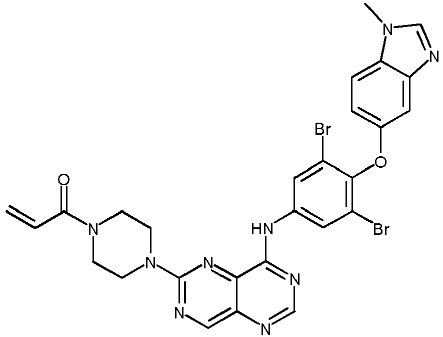 |
| I-10 | 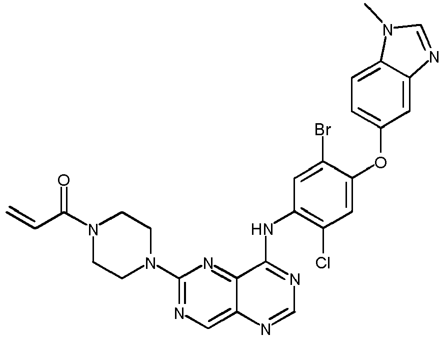 |
| I-11 | 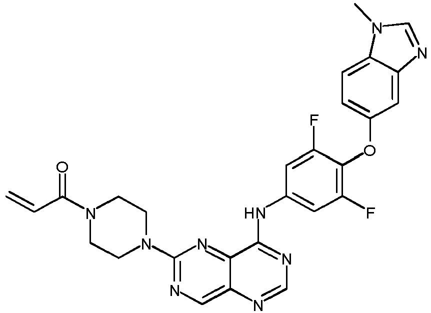 |
| I-12 | 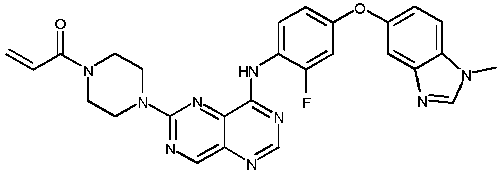 |

11. Composé selon l'une quelconque des revendications 1 à 3, 6 ou 8 à 10 ayant la structure suivante :

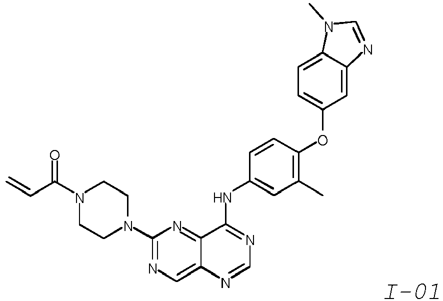

12. Sel pharmaceutiquement acceptable du composé selon l'une quelconque des revendications 1 à 3, 6 ou 8 à 10 ayant la structure suivante :

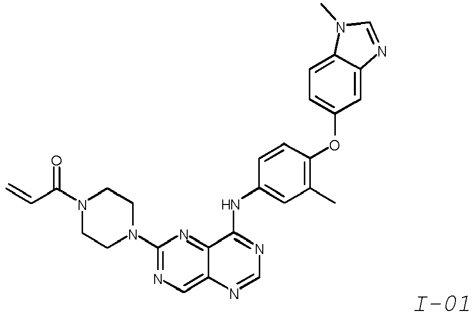

13. Composition pharmaceutique comprenant une quantité thérapeutiquement efficace d'au moins un composé de formule **(I)** selon l'une quelconque des revendications 1 à 12 ou d'un sel pharmaceutiquement acceptable de celui-ci et un ou plusieurs excipients pharmaceutiquement acceptables.

14. Composé selon une ou plusieurs des revendications 1 à 12, ou sel pharmaceutiquement acceptable de celui-ci pour son utilisation comme médicament.

15. Composé selon une ou plusieurs des revendications 1 à 12 pour son utilisation dans le traitement d'un cancer du cerveau, d'un cancer du sein, d'un cancer des voies biliaires, d'un cancer de la vessie, d'un cancer du col de l'utérus, d'un cancer colorectal, d'un cancer de l'endomètre, d'un cancer de la peau, d'une tumeur de l'œsophage, d'une tumeur de la tête et du cou, d'un cancer gastro-intestinal, d'une tumeur de la vésicule biliaire, d'un cancer du rein, d'un cancer du foie, d'un cancer du poumon ou d'un cancer de la prostate.
